# EUROPEAN PATENT SPECIFICATION

(11) **EP 2 331 110 B1**
(45) Date of publication and mention of the grant of the patent: **28.08.2013**
(21) Application number: 09736339.4
(22) Date of filing: 25.09.2009
(51) Int. Cl.: A61K 35/74, A61P 31/04, A61P 31/12

(54) **COMPOSITIONS AND METHODS FOR ENGINEERING PROBIOTIC YEAST**
ZUSAMMENSETZUNGEN UND VERFAHREN ZUR GENTECHNISCHEN HERSTELLUNG EINER PROBIOTISCHEN HEFE
COMPOSITIONS ET PROCÉDÉS POUR MODIFIER UNE LEVURE PROBIOTIQUE

(30) Priority: 25.09.2008 US 194161 P
(43) Date of publication of application: 15.06.2011
(73) Proprietor: Glycosyn LLC, Waltham, MA 02451 (US)
(72) Inventor: MCCOY, John, M., Reading, MA 01867 (US); ROBBINS, Phillips, W., Newburyport, MA 01950 (US)
(74) Representative: Mintz Levin Cohn Ferris Glovsky and Popeo LLP
(86) International application number: PCT/US2009/058389
(87) International publication number: WO 2010/036898

(56) References cited:
- WO-A2-00/29603
- WO-A2-02/00879
- WO-A2-2005/055944

## Description

### FIELD OF THE INVENTION

The invention provides compositions and methods for engineering probiotic yeast that prevent infections through inhibition of pathogen adherence to epithelia.

### BACKGROUND OF THE INVENTION

Worldwide, infectious diarrhea is responsible for approximately 20% of all mortality in children under the age of 5, and for an estimated 2.5 millions deaths annually. In the United States, infectious diarrhea has an annual incidence of over 200 million cases and is responsible for approximately 900,000 hospitalizations and 5,000 deaths per year. Included in these numbers are 200,000 children under five years of age hospitalized in the U.S. each year with active diarrheal disease, accounting for nearly 880,000 inpatient days, over 500 deaths, and almost one billion dollars of inpatient cost.

WO 2005/055944 describes oligosaccharide compositions and their use in the treatment of infections, such as compositions comprising a fucose group in an α 1,2 linkage, an α 1,3 linkage or an α 1,4 linkage to a galactose group.

WO 02/00879 relates to methods and compositions to modify eukaryotic microorganisms genetically in order to produce glycosylated proteins.

WO 00/29603 relates to the use of cells to express glycosyltransferases, including a microorganism producing a nucleotide sugar and a recombinant glycosyltransferase, namely α(1,2) galactosyltransferase.

### SUMMARY OF THE INVENTION

There is herein described compositions comprising a yeast cell, engineered to produce α(1,2) fucosylated glycans on its cell surface, in a probiotic formulation that promotes intestinal health by decreasing the adherence and or colonization of the gastrointestinal tract by pathogenic organisms. In accordance with the invention there is provided a composition comprising a yeast cell, said yeast cell comprising an exogenous nucleic acid molecule encoding GDP-mannose-4,6-dehydratase (GMD) and an exogenous nucleic acid molecule encoding GDP-L-fucose synthetase

(GFS), wherein said yeast cell further comprises a nucleic acid molecule encoding GDP-mannose pyrophosphorylase, phosphomannose isomerase, or phosphomannose mutase. Any edible yeast cell is suitable host for expression of the glycans. Preferably, the genus of the yeast cell is *Kluyveromyces. Kluyveromyces lactis* is tolerant to conditions found in the human gut and may be particularly well suited to probiotic applications, although yeast of other genera could be used. For example, Saccharomyces yeast cells are used to display α(1,2) fucosylated glycans on the cell surface of a yeast cell. In one example, the formulation also contains probiotic bacteria such as *Lactobacilli;* alternatively, the formulation does not comprise bacterial probiotic strains.

The engineered yeast cell harbours a exogenous (recombinant) nucleic acid molecule encoding GDP-mannose-4,6-dehydratase (GMD) and an exogenous (recombinant) nucleic acid molecule encoding GDP-L-fucose synthetase (GFS). In one aspect, the GMD is selected from the group consisting of *H. pylori* GMD, *E. coli* GMD, and *Homo sapiens* GMD, and the GFS is selected from the group *H. pylori* GFS, *E. coli* GFS, and *Homo sapiens* GFS. The composition further comprises a recombinant nucleic acid construct encoding GDP-mannose pyrophosphorylase (e.g. *K.lactis* PSA1) and directing increased expression of this enzyme, recombinant nucleic acid construct encoding phosphomannose isomerase (e.g. *K.lactis* locus KLLAOD13728g, PMI) or phosphomannose mutase (e.g. *K.lactis* SEC53, PMM), leading to increased expression of these enzymes.

The invention also provides for a yeast cell that further harbors a nucleic acid molecule encoding a nucleotide sugar transporter (NST) (to ensure that the appropriate precursor molecules synthesized in the cell cytoplasm are made available in the golgi for incorporation into cell-surface glycans). In one aspect, the NST is selected from the group consisting of human FUCT1 (GDP-fucose transporter), mouse SLC35a2 (UDP-galactose transporter), human UGT1 (UDP-galactose transporter), *Schizosaccharomyces pombe* GMS1 (UDP-galactose transporter), *Saccharomyces cerevisiae* HUT1 (UDP-galactose transporter). Optionally, the yeast cell over-expresses an endogenous NST.

In another aspect, the yeast cell lacks terminal *N*-acetylglucosaminyltransferase activity or UDP-*N*-acetylglucosamine transporter activity. Preferably, the yeast cell is a *Kluyveromyces lactis* yeast cell carrying mutations in the GNT1 (MNN2-1) *(N-*acetylglucosaminyltransferase) or *MNN2* (MNN2-2) (UDP-*N-*acetylglucosamine transporter) genes.

The invention also provides a yeast cell that further harbors a nucleic acid molecule encoding an α(1,2) galactosyltransferase. Optionally, the nucleic acid molecule encoding an α(1,2) galactosyltransferase is *Schizosaccharomyces pombe GMA12.*

In another aspect, the yeast cell further harbors a nucleic acid molecule encoding a β(1,3) galactosyltransferase. Optionally, the nucleic acid molecule encoding a β(1,3) galactosyltransferase is *Schizosaccharomyces pombe* PVG3.

The invention also provides for a yeast cell that further harbors a recombinant nucleic acid molecule encoding a fucosyltransferase. Preferably, the fucosyltransferase is a human α(1,2) fucosyltransferase (FUT1 or FUT2).

Preferably, the yeast cell displays α(1,2) fucosylated glycans on its cell surface. More preferably, the yeast cell is a *Kluyveromyces lactis* yeast cell.

Exemplary protein (amino acid) sequences and GENBANK™ Accession Numbers, the listings of which also contain nucleic acid sequences encoding the reference protein are provided below.

H. pylori GMD:
>YP_003056882 GDP-mannose 4,6-dehydratase (GDP-D-mannose dehydratase)
[Helicobacter pylori B38].
MKEKIALITGVTGQDGSYLAEYLLNLGYEVHGLKRRSSSINTSRIDHLYEDLHSDHKRRF
FLHYGDMTDSSNLIHLIATTKPTEIYNLAAQSHVKVSFETPEYTANADGIGTLRILEAMR
ILGLEKKTRFYQASTSELYGEVLETPQNENTPFNPRSPYAVAKMYAFYITKNYREAYNLF
AVNGILFNHESRVRGETFVTRKITRAASTIAYNLTDCLYLGNLDAKRDWGHAKDYVKMMH
LMLQAPTPQDYVIATGKTTSVRDFVKMSFEFIGINLEFQNTGIKEIGLIKSVDEKRANAL
QLNLSHLKTGQIVVRIDEHYFRPTEVDLLLGDPTKAEKELGWVREYDLKELVKDMLEYDL
KECQKNLYLQDGGYILRNFYE (SEQ ID NO:1)

E. coli GMD:
>AAL67553 GDP-mannose dehydratase Gmd [Escherichia coli].
MTKVALITGVTGQDGSYLAEFLLEKGYEVHGIKRRASSFNTERVDHIYQDPHSAKPNFHL
HYGDLTDTSNITRILQEVKPDEVYNLGAMSHVAVSFESPEYTADVDAIGTLRLLEAIRIL
GLEKKTRFYQASTSELYGLVQEIPQKETTPFYPRSPYAVAKLYAYWITVNYRESYGIYAC
NGILFNHESPRRGETFVTRKITRAIANIAQGLESCLYLGNMDSLRDWGHAKDYVRMQWMM
LQQEQPEDFVIATGVQYSVREFVEMTAEQLGIKLSFEGKGVDEKGIVVSVTGDKAPGVKT
GDVIVAVDPRYFRPAEVETLLGDPAKAHEKLGWKPEITLREMISEMVANDLQTAKKHVLL
KSHGFNANLAQE (SEQ ID NO:2)

Homo sapiens GMD:
>EAW55075 GDP-mannose 4,6-dehydratase [Homo sapiens].
MAHAPARCPSARGSGDGEMGKPRNVALITGITGQDGSYLAEFLLEKGYEVHGIVRRSSSF
NTGRIEHLYKNPQAHIEGNMKLHYGDLTDSTCLVKIINEVKPTEIYNLGAQSHVKISFDL
AEYTADVDGVGTLRLLDAVKTCGLINSVKFYQASTSELYGKVQEIPQKETTPFYPRSPYG
AAKLYAYWIVVNFREAYNLFAVNGILFNHESPRRGANFVTRKISRSVAKIYLGQLECFSL
GNLDAKRDWGHAKDYVEAMWLMLQNDEPEDFVIATGEVHSVREFVEKSFLHIGKTIVWEG
KNENEVGRCKETGKVHVTVDLKYYRPTEVDFLQGDCTKAKQKLNWKPRVAFDELVREMVH
ADVELMRTNPNA (SEQ ID NO:3)

H. pylori GFS:
>AAC64910 O-antigen biosynthesis protein [Helicobacter pylori].
MNEIILITGAYGMVGQNTALYFKKNKPDVTLLTPKKSELCLLDKDNVQAYLKEYKPTGII
HCAGRVGGIVANMNDLSTYMVENLLMGLYLFSSALDLGVKKAINLASSCAYPKYAPNPLK
ESDLLNGSLEPTNEGYALAKLSVMKYCEYVSTEKGGFYKTLVPCNLYGEFDKFEEKIAHM
IPGLIARMHTAKLKNEKNFAMWGDGTARREYLNAKDLARFIALAYENIASMPSVMNVGSG
VDYSIEEYYEMVAQVLDYKGVFVKDLSKPVGMQQKLMDISKQKALKWELEIPLEQGIKEA
YEYYLKLLEV (SEQ ID NO: 4)

E. coli GFS:
>AAC77843 GDP-L-fucose synthetase [Escherichia coli].
MSKQRVFIAGHRGMVGSAIRRQLEQRGDVELVLRTRDELNLLDSRAVHDFFASERIDQVY
LAAAKVGGIVANNTYPADFIYQNMMIESNIIHAAHQNDVNKLLFLGSSCIYPKLAKQPMA
ESELLQGTLEPTNEPYAIAKIAGIKLCESYNRQYGRDYRSVMPTNLYGPHDNFHPSNSHV
IPALLRRFHEATAQNAPDVVVWGSGTPMREFLHVDDMAAASIHVMELAHEVWLENTQPML
SHINVGTGVDCTIRDVAQTIAKVVGYKGRVVFDASKPDGTPRKLLDVTRLHQLGWYHEIS
LEAGLASTYQWFLENQDRFRG (SEQ ID NO:5)

Homo sapiens GFS:
>Q13630 GDP-L-fucose synthetase [Homo sapiens]
MGEPQGSMRILVTGGSGLVGKAIQKVVADGAGLPGEDWVFVSSKDADLTDTAQTRALFEK
VQPTHVIHLAAMVGGLFRNIKYNLDFWRKNVHMNDNVLHSAFEVGARKVVSCLSTCIFPD
KTTYPIDETMIHNGPPHNSNFGYSYAKRMIDVQNRAYFQQYGCTFTAVIPTNVFGPHDNF
NIEDGHVLPGLIHKVHLAKSSGSALTVWGTGNPRRQFIYSLDLAQLFIWVLREYNEVEPI
ILSVGEEDEVSIKEAAEAVVEAMDFHGEVTFDTTKSDGQFKKTASNSKLRTYLPDFRFTP
FKQAVKETCAWFTDNYEQARK (SEQ ID NO:6)

K.lactis PSA1:
>Q70SJ2 GDP-mannose pyrophosphorylase [Kluyveromyces lactis].
MKGLILVGGYGTRLRPLTLTVPKPLVEFGNRPMILHQIEALAAAGVTDIVLAVNYRPEVM

VETLKKYEDEFGVSITFSVETEPLGTAGPLKLAESVLKKDNSPFFVLNSDVICDYPFKEL
ADFHQAHGGKGTIVATKVDEPSKYGVIVHDIATPNLIDRFVEKPVEFVGNRINAGLYILN
PEVIDLIDLKPTSIEKETFPILVEQKSLYSFDLEGYWMDVGQPKDFLSGTVLYLNSLSKR
DPAKLAKGENIVGNVLVDPTAKISPTAKVGPDVVIGPNVVIGDGVRITRSVALSNSHIKD
HALVKSTIIGWNSTVGKWARLEGVTVLGDDVEVKDEIYINGGKVLPHKSISVNVPKEAII
M (SEQ ID NO:7)

K.lactis locus  KLLA0D13728g, PMI:
>CAH00771 KLLA0D13728p phosphomannose isomerase [Kluyveromyces lactis].
MPQLFRLDAGFQQYDWGKIGSSSAVAQFAAHSDPSVKIDEQKPYAELWMGTHHKVPSYNH
DTKESLRDLIEADPVGMLGQGNVDKFGSMKELPFLFKVLSIKKVLSIQAHPDKALAKVLH
FNDPANYPDDNHKPEMALAVTDFEGFCGFKPLEEIADELQRIPELRNIVGDEVSETFINN
INPEAVKDSADDAKNKKLLQQVFSKVMNASDKVVVENARALIKRAHESPADFNKDTLPQL
LIDLNEQFPDDVGLFCGGLLLNHCNLKAGEAIFLRAKDPHAYISGDIIECMAASDNVVRA
GFTPKFKDVKNLVEMLTYTYDSVEKQKMSPENFERSSGQGKSVLFNPPIEEFAVLYTTFQ
DGVGTRHFEGLHGPSIVITTKGNGFIKTGDLKLKAEPGFVFFIAPGTEVDFIADDTDFTT
YRAFVEPN (SEQ ID NO:8)

K.lactis SEC53, PMM:
>CAD21466 phosphomannomutase [Kluyveromyces lactis].
MSVSEFSHKEQPTTLVLFDVDGTLTPARLTVSDEVRDILKRLREKVVIGFVGGSDLSKQL
EQLGSDVLDQFDYAFSENGLTAYRLGKELASQSFIEWIGEEEYNKLAKFILQYLASIDLP
KRRGTFLEFRNGMINVSPIGRNASTAERNEFEQFDKEHQVRAKFVEALKKEFAHLSLTFS
IGGQISFDVFPTGWDKTYCLRHVEADGFKEIHFFGDKTYKGGNDYEIYVDDRTIGHSVES
PADTVRILKELFDL (SEQ ID NO:9)

human FUCT1 (GDP-fucose transporter):
>Q96A29 human GDP-fucose transporter FUCT1 GDP-fucose transporter 1 (Solute
carrier family 35 member C1) [Homo sapiens].






 (SEQ ID NO:10)

mouse SLC35a2 (UDP-galactose transporter):
>CAM24549 solute carrier family 35 (UDP-galactose transporter) member 2
[Mus musculus].
MAAVGVGGSTAAAGAGAVSSGALEPGSTTAAHRRLKYISLAVLVVQNASLILSIRYARTL
PGDRFFATTAVVMAEVLKGLTCLLLLFAQKRGNVKHLVLFLHEAVLVQYVDTLKLAVPSL
IYTLQNNLQYVAISNLPAATFQVTYQLKILTTALFSVLMLNRSLSRLQWASLLLLFTGVA
IVQAQQAGGSGPRPLDQNPGAGLAAVVASCLSSGFAGVYFEKILKGSSGSVWLRNLQLGL
FGTALGLVGLWWAEGTAVASQGFFFGYTPAVWGVVLNQAFGGLLVAVVVKYADNILKGFA
TSLSIVLSTVASIRLFGFHLDPLFALGAGLVIGAVYLYSLPRGAVKAIASASASGPCIHQ
QPPGQPPPPQLSSRGDLTTEPFLPKLLTKVKGS (SEQ ID NO:11)

human UGT1 (UDP-galactose transporter):
>BAA12673 human UDP-galactose transporter UGT1UDP-galactose translocator
[Homo sapiens].






 (SEQ ID NO:12)

Schizosaccharomyces pombe GMS1 (UDP-galactose transporter):
>P87041 S.pombe UDP-galactose transporter Gms1 UDP-galactose transporter
[Schizosaccharomyces pombe].
MAVKGDDVKWKGIPMKYIALVLLTVQNSALILTLNYSRIMPGYDDKRYFTSTAVLLNELI
KLVVCFSVGYHQFRKNVGKEAKLRAFLPQIFGGDSWKLAIPAFLYTCQNNLQYVAAGNLT
AASFQVTYQLKILTTAIFSILLLHRRLGPMKWFSLFLLTGGIAIVQLQNLNSDDQMSAGP
MNPVTGFSAVLVACLISGLAGVYFEKVLKDTNPSLWVRNVQLSFFSLFPCLFTILMKDYH
NIAENGFFFGYNSIVWLAILLQAGGGIIVALCVAFADNIMKNFSTSISIIISSLASVYLM
DFKISLTFLIGVMLVIAATFLYTKPESKPSPSRGTYIPMTTQDAAAKDVDHKH (SEQ ID NO:13)

Saccharomyces cerevisiae HUT1 (UDP-galactose transporter):
> NP_015080 S.cerevisiae UDP-galactosetransporter Hut1 Hut1p [Saccharomyces
cerevisiae].
MAGSTSSLVICAIGIYATFLTWALVQEPLATRTWPNSMGKFQFPNVISLIQASVAMMMGY
LYLNWKKVEYPPRKMIKDHWKQLMLISFTQSSSGPLATTSLKHVDYLTYMLAKSCKMIPV
LLVHLLLYRTPIASQKKVVALLVSLGVTIFTIGGNDGKKLKRSFNESGNDNKLQGFGLLF
SSLFLDGLTNATQDKLLKANKAKEKGKQTLITGAHLMFTLNLFVILWNILYFIVIDCKQW
DNAVSVLTMDPQVWGYLMLYSFCGAMGQCFIFYTLEQFGSLVLIMITVTRKMVSMILSII
VFGKSVRFQQWVGMFIVFGGITWEALNKKKANIPKAKSA (SEQ ID NO:14)

K.lactis GNT1 (MNN2-1) (N-acetylglucosaminyltransferase):
>AAD25740 alphaN-acetylglucosamine transferase [Kluyveromyces lactis].







 (SEQ ID NO:15)

K.lactis MNN2 (MNN2-2) (UDP-N-acetylglucosamine transporter):
>XP_451673 MNN2_KLULA UDP-N-acetylglucosamine transporter [Kluyveromyces
lactis].
MSFVLILSLVFGGCCSNVISFEHMVQGSNINLGNIVTFTQFVSVTLIQLPNALDFSHFPF
RLRPRHIPLKIHMLAVFLFFTSSVANNSVFKFDISVPIHIIIRCSGTTLTMIIGWAVCNK
RYSKLQVQSAIIMTLGAIVASLYRDKEFSMDSLKLNTDSVGMTQKSMFGIFVVLVATALM
SLLSLLNEWTYNKCGKHWKETLFYSHFLALPLFMLGYTRLRDEFRDLLISSDSMDIPIVK
LPIATKLFMLIANNVTQFICIKGVNMLASNTDALTLSVVLLVRKFVSLLLSVYIYKNVLS
VTAYLGTITVFLGAGLYSYGSVKTALPR (SEQ ID NO:16)

Schizosaccharomyces pombe GMA12:
>CAA83200 S.pombe alpha-1,2galactosyltransferase Gma12 alpha-1,2-
galactosyltransferase [Schizosaccharomyces pombe].
MRFAPYLISAVVITTIILGGAWWTSAMDTKLQTKMKEIIDQHTSTWTPVVSSVTSTQTDT
LRVTISEVVSVTATLTETFTATPTVTSVVHALATTDPHPDNSKIVILMGSNFQNDANSPL
HPFAQSIIKNRREYAERHGYKFEFLDADAYASRVTGHLMPWVKVPMLQDTMKKYPDAEWI
WWLDHDALVMNKDLNVVDHVLKHDRLNTILTREAEYKSGAGIPADGFRTPKDQDAKDVHF
IISQDFNGINAGSLFIRNSEVGRWIVDLWFEPLYLDHIQGYAEQQAFSHMVFYHPQVYKH
VGVVPLKAINAYDFDDNIWGYDDGDLCIHFAGCNYFKNCPEKFLKYAQILSSKQGSDWMS
AQEKDHIQNLLKPSS (SEQ ID NO:17)

Schizosaccharomyces pombe PVG3:
>CAB58972 S.pombe beta-1,3galactosyltransferase Pvg3 beta-1,3-
galactosyltransferase (PMID 15173185) [Schizosaccharomyces pombe].
MFSNSKKKIFLYVLIAGVATFSFAFLVLNRLQAEEHSLAYVENLFLDPFIKQNESLAHAN
DRPFKLYLGIFSQAKNVDRRNFLRTDYNEYIKEFAVNDTVDVRFILGLPENEQELATIRE
EQRTYGDLAVLPIPENVDAGKSIVYFQTFLEGYQPFPLFSELADNLIMPSTQFHGSFIYN
QSIKTYELPGMKEFQDLGEPKHDYDFIVKADDDSFLNLPRLFEMLKEHVGKSRFYFGRDC
TRRELPTAVRDFPYMCGFFYIVSPDMAYEVAKRRNIIIPFEDAQTGYSIYLSGNVKNAEF
SKCTLYDLILPNEGFNYRQSYLRIDAIAVHKLKSIPLLSTVSNWFKKMYEHRANCSALIE
TERLSCLQATIPLPSLDV (SEQ ID NO:18)

human (1,2) fucosyltransferase FUT1:
>AAH74732 humanalpha-1,2fucosyltransferase FUT1Fucosyltransferase 1 [Homo sapiens].
MWLRSHRQLCLAFLLVCVLSVIFFLHIHQDSFPHGLGLSILCPDRRLVTPPVAIFCLPGT
AMGPNASSSCPQHPASLSGTWTVYPNGRFGNQMGQYATLLALAQLNGRRAFILPAMHAAL
APVFRITLPVLAPEVDSRTPWRELQLHDWMSEEYADLRDPFLKLSGFPCSWTFFHHLREQ
IRREFTLHDHLREEAQSVLGQLRLGRTGDRPRTFVGVHVRRGDYLQVMPQRWKGVVGDSA
YLRQAMDWFRARHEAPVFVVTSNGMEWCKENIDTSQGDVTFAGDGQEATPWKDFALLTQC
NHTIMTIGTFGFWAAYLAGGDTVYLANFTLPDSEFLKIFKPEAAFLPEWVGINADLSPLW
TLAKP (SEQ ID NO:19)

human (1,2) fucosyltransferase FUT2:
>NP_001091107 human alpha-1,2fucosyltransferase FUT2 fucosyltransferase 2
[Homo sapiens].
MLVVQMPFSFPMAHFILFVFTVSTIFHVQQRLAKIQAMWELPVQIPVLASTSKALGPSQL
RGMWTINAIGRLGNQMGEYATLYALAKMNGRPAFIPAQMHSTLAPIFRITLPVLHSATAS
RIPWQNYHLNDWMEEEYRHIPGEYVRFTGYPCSWTFYHHLRQEILQEFTLHDHVREEAQK
FLRGLQVNGSRPGTFVGVHVRRGDYVHVMPKVWKGVVADRRYLQQALDWFRARYSSLIFV
VTSNGMAWCRENIDTSHGDVVFAGDGIEGSPAKDFALLTQCNHTIMTIGTFGIWAAYLTG
GDTIYLANYTLPDSPFLKIFKPEAAFLPEWTGIAADLSPLLKH (SEQ ID NO:20)

H.pylori futC:
>AAD29869 alpha-1,2-fucosyltransferase [Helicobacter pylori].
MAFKVVQICGGLGNQMFQYAFAKSLQKHSNTPVLLDITSFDWSDRKMQLELFPINLPYAS
AKEIAIAKMQHLPKLVRDALKCMGFDRVSQEIVFEYEPELLKPSRLTYFYGYFQDPRYFD
AISPLIKQTFTLPPPPENNKNNNKKEEEYHRKLSLILAAKNSVFVHIRRGDYVGIGCQLG
IDYQKKALEYMAKRVPNMELFVFCEDLEFTQNLDLGYPFMDMTTRNKEEEAYWDMLLMQS
CQHGIIANSTYSWWAAYLIENPEKIIIGPKHWLFGHENILCKEWVKIESHFEVKSQKYNA (SEQ ID NO:21)

The invention provides for a method for producing yeast containing GDP-fucose comprising culturing the yeast harbouring a recombinant nucleic acid molecule encoding GDP-mannose-4,6-dehydratase (GMD) and a recombinant nucleic acid molecule encoding GDP-L-fucose synthetase (GFS), wherein said yeast cell further comprises a nucleic acid molecule encoding GDP-mannose pyrophosphorylase, phosphomannose isomerase or phosphomannose mutase, under conditions in which the encoded GDP-mannose-4,6-dehydratase and the encoded GDP-L-fucose synthetase are expressed.

Also provided is a method for producing yeast that are competent for the Golgi import of both GDP-fucose and UDP-galactose comprising culturing the yeast that further harbours a nucleic acid molecule encoding a nucleotide sugar transporter under conditions in which the encoded nucleotide sugar transporter is expressed.

The invention also provides a method for producing yeast that lack terminal α(1,2) GlcNAc mannans comprising introducing the *Kluyveromyces lactis* yeast cell *mnn2-1* or *mnn2-2* mutation into the yeast cell that further harbors a nucleic acid molecule encoding a nucleotide sugar transporter.

Also provided is a method of producing yeast with α(1,2) galactose residues on surface polymannose, comprising culturing the yeast that further harbors a nucleic acid molecule encoding an α(1,2) galactosyltransferase under conditions in which the encoded α(1,2) glycosyltransferase is expressed.

The invention also provides a method of producing yeast with β(1,3) galactose linked to surface α(1,2) galactose residues comprising culturing the yeast that further harbors a nucleic acid molecule encoding a β(1,3) galactosyltransferase under conditions in which the β(1,3) glycosyltransferase is expressed.

A method of producing yeast with α(1,2) fucosylated glycans on its cell surface comprising is carried out by culturing the yeast that further harbors a nucleic acid molecule encoding a fucosyltransferase under conditions in which the fucosyltransferase is expressed.

The disclosure also provides for a method of preventing, treating, or reducing an infection, or the risk of developing an infection in a mammalian subject (human or animal) comprising administering a yeast cell that displays α(1,2) fucosylated glycans on its cell surface to a subject. The subject is infected with or at risk of becoming infected with a pathogen that binds to a fucosylated glycan. The glycans inhibit or reduce binding of a pathogen to the cells of the subject and thereby reduce infection. In one aspect, the infection is caused by a norovirus such as Norwalk-like virus, or strains of bacteria from genera including, but not limited to, *Campylobacter* (e.g. *C. jejuni), Escherichia* (e.g. *E.coli), Salmonella* (e.g. *S. enterica) Vibrio* (e.g. *V.cholerae),* or *Helicobacter* (e.g. *H.pylori).* The therapeutic compositions are useful not only for humans but also for prophylactic and therapeutic intervention to prevent and/or inhibit binding and infection of pathogens in livestock or companion animals such as pigs, horses, chickens, cows, sheep, goats, dogs, or cats. The formulations are suitable for administration to wild and domesticated animals.

Optionally, the disclosure features a vector, *e.g.*, a vector containing a nucleic acid. The vector can further include one or more regulatory elements, *e.g.,* a heterologous promoter or elements required for translation in yeast. The regulatory elements can be operably linked to a fusion protein in order to express the fusion protein. In yet another aspect, the disclosure features an isolated recombinant cell, *e.g.,* a yeast cell containing an aforementioned nucleic acid molecule or vector. The nucleic acid sequence is optionally integrated into the genome. The sequence of an exemplary *K.lactis* expression vector is provided below; the vector includes two *K.lactis* promoter sequences "s2" and "s3" in the expression plasmid which possess strong promoter activity.

K.lactis expression vector pEKs2 3deltaU-fcl-gmd

tttaaacGCTTTTTCTTTCCAATTTTTTTTTTTTCGTCATTATAGAAATCATTACGACCG
AGATTCCCGGGTAATAACTGATATAATTAAATTGAAGCTCTAATTTGTGAGTTTAGTATA
CATGCATTTACTTATAATACAGTTTTTTAGTTTTGCTGGCCGCATCTTCTCAAATATGCT
TCCCAGCCTGCTTTTCTGTAACGTTCACCCTCTACCTTAGCATCCCTTCCCTTTGCAAAT
AGTCCTCTTCCAACAATAATAATGTCAGATCCTGTAGAGACCACATCATCCACGGTTCTA
TACTGTTGACCCAATGCGTCTCCCTTGTCATCTAAACCCACACCGGGTGTCATAATCAAC
CAATCGTAACCTTCATCTCTTCCACCCATGTCTCTTTGAGCAATAAAGCCGATAACAAAA
TCTTTGTCGCTCTTCGCAATGTCAACAGTACCCTTAGTATATTCTCCAGTAGCTAGGGAG
CCCTTGCATGACAATTCTGCTAACATCAAAAGGCCTCTAGGTTCCTTTGTTACTTCTTCC
GCCGCCTGCTTCAAACCGCTAACAATACCTGGGCCCACCACACCGTGTGCATTCGTAATG
TCTGCCCATTCTGCTATTCTGTATACACCCGCAGAGTACTGCAATTTGACTGTATTACCA
ATGTCAGCAAATTTTCTGTCTTCGAAGAGTAAAAAATTGTACTTGGCGGATAATGCCTTT
AGCGGCTTAACTGTGCCCTCCATGGAAAAATCAGTCAAGATATCCACATGTGTTTTTAGT
AAACAAATTTTGGGACCTAATGCTTCAACTAACTCCAGTAATTCCTTGGTGGTACGAACA
TCCAATGAAGCACACAAGTTTGTTTGCTTTTCGTGCATGATATTAAATAGCTTGGCAGCA
ACAGGACTAGGATGAGTAGCAGCACGTTCCTTATATGTAGCTTTCGACATGATTTATCTT
CGTTTCCTGCAGGTTTTTGTTCTGTGCAGTTGGGTTAAGAATACTGGGCAATTTCATGTT
TCTTCAACAgtcgaCTGTGCTCCTTCCTTCGTTCTTCCTTCTGCTCGGAGATTACCGAAT
CAAAAAAATTTCAAAGAAACCGGAATCAAAAAAAAGAACAAAAAAAAAAAAGATGAATTG
AAAAGCgcGGCCGtGCACAAACGAACGTCTCACTTAATCTTCTGTACTCTGAAGAGGAGT
GGGAAATACCAAGAAAAACATCAAACTCGAATGATTTTCCCAAACCCCTACCACAAGATA
TTCATCAGCTGCGAGATAGGCTGATCAGGAGCAAGCTCGTACGAGAAGAAACAAAATGAC
AAAAAAAATCCTATACTATATAGGTTACAAATAAAAAAGTATCAAAAATGAAGCCTGCAT
CTCTCAGGCAAATGGCATTCTGACATCCTCTTGAGgatctCTCGAGTTTATGACTCCAGC
GCGATCGCCACGTCGTAGCCGTGAGATTTCAGCAGAGAGTGTTTTTTCGCCGCTTCGAGG
TCATTAGCCACCATTTCAGACACCATCTCTCTGAGGGTGATTTCCGGTTTCCAGCCCAGT
TTTTCGTGCGCTTTGGTCGGGTCGCCGAGCAGCGTTTCAACTTCAGCCGGACGGAAGTAA
CGCGGGTCAACAGCGATAATCACATCACCCGGTTTAACGCCCGGCGCGTCATGCCCGGTG
ACGGAAACCACAATGCCCTTCTCTTCAACGCCCGTGCCTTCAAAGCGCAGTTTGATGCCC
AGCTGTGCTGCCGCCATTTCCACGAACTGACGCACGGAGTACTGAACGCCGGTCGCGATA
ACGAAATCTTCCGGCTGTTCCTGCTGCAGCATCATCCACTGCATTTTTACGTAGTCTTTG
GCGTGGCCCCAGTCACGCAGGGAATCCATATTGCCGAGGTACAGGCACGACTCCAGCCCC
TGGGCGATGTTGGCGATTGCGCGGGTGATTTTGCGGGTAACGAAGGTTTCGCCGCGGCGC
GGGGATTCATGGTTGAAGAGAATTCCGTTACAGGCGTACATGCCGTAGGATTCACGGTAG
TTAACGGTGATCCAGTAGGCGTACAGTTTGGCGACCGCATACGGAGATCGCGGGTAGAAC
GGCGTGGTCTCTTTCTGCGGAATTTCCTGCACCAGACCATACAGTTCAGAGGTGGAAGCC
TGATAGAAACGAGTTTCTTTTCCAGACCGAGGAAGCGGATCGCCTCCAGCAGGCGCAGC
GTACCCATCGCGTCGACGTCAGCGGTATATTCTGGTGACTCAAAAGAGACCGCAACGTGG
CTCATTGCGCCCAGGTTGTACACTTCATCCGGCTGTACTTCACGCAAAATGCGCGTCAGG
TTAGAGGTATCACTCAGGTCGCCATAATGCAGATGGAATTTCGGGTTGCAGGTGTGCGGA
TCCTGATAAATGTGATCCACGCGCTCGGTGTTGAATGACGATGCGCGACGCTTAATACCA
TGCACCTCGTAACCTTTTTCCAGCAGAAACTCTGCCAGGTAAGAACCGTCTTGTCCGGTT
ACACCGGTGATGAGAGCGACTTTTGACATTTTTTTCAAGCTTactagtGGATCCAGATCA
GCAGTAGCTCTCTGTGCCTCTTTTCCTCTATCCTGTTTTATGCAAAATGTGCTATCGTAA
TGGTAAAATTCAATGGCTTAAAGGTTGAAATTTTATTTCAAAAGATTCACAGCTTTTCCT
CTTTACAATGTAAAAATTTTTTTTTCTTCTTTCGTGTATATTGAGAAGATTGACATGAT
GGTCAAGTTTATGACGAGATGAGATGCGATGAGGGGAAAAAGAGAGGATCTGCGGCCGt
GCACAAACGAACGTCTCACTTAATCTTCTGTACTCTGAAGAGGAGTGGGAAATACCAAGA
AAAACATCAAACTCGAATGATTTTCCCAAACCCCTACCACAAGATATTCATCAGCTGCGA
GATAGGCTGATCAGGAGCAAGCTCGTACGAGAAGAAACAAAATGACAAAAAAAATCCTAT
ACTATATAGGTTACAAATAAAAAAGTATCAAAAATGAAGCCTGCATCTCTCAGGCAAATG
GCATTCTGACATCCTCTTGAGgatctctcgagtttaCCCCCGAAAGCGGTCTTGATTCTC
AAGGAACCACTGGTAAGTGCTGGCAAGCCCCGCTTCCAGTGAGATTTCGTGATACCAGCC
AAGCTGATGCAGGCGCGTCACATCCAGCAGTTTGCGCGGCGTGCCATCCGGTTTGCTGGC
ATCAAAAACCACCCGGCCTTTGTAACCCACCACTTTGGCGATGGTTTGCGCCAGCTCGCG

GATAGTGCAGTCAACGCCCGTGCCGACGTTAATGTGCGACAACATCGGCTGGGTGTTCTC
CAGCCAGACTTCATGCGCCAGCTCCATGACATGAATGCTCGCCGCCGCCATATCATCGAC
GTGCAGAAATTCGCGCATCGGTGTACCGCTGCCCCATACCACCACGTCCGGCGCATTCTG
TGCCGTCGCCTCGTGGAAGCGACGCAGCAATGCTGGGATCACATGCGAATTACTCGGGTG
GAAGTTGTCGTGTGGCCCGTACAGGTTGGTCGGCATGACTGAGCGGTAATCGCGTCCGTA
CTGGCGGTTGTATGATTCGCACAGTTTGATCCCGGCGATTTTGGCAATAGCATAAGGCTC
GTTAGTCGGCTCCAGCGTGCCCTGCAACAACTCGCTTTCTGCCATCGGCTGTTTTGCCAG
TTTCGGGTAGATGCAGGACGATCCGAGAAACAGCAGTTTGTTCACGTCGTTCTGATGCGC
GGCGTGAATGATGTTGCTCTCAATCATCATGTTCTGGTAGATGAAATCCGCCGGATAGGT
GTTGTTGGCAACAATGCCGCCCACTTTCGCCGCCGCCAGATAGACCTGGTCAATACGTTC
GCTGGCAAAGAAATCATGCACGGCGCGGCTGTCCAGCAGGTTCAGCTCGTCGCGGGTGCG
TAATACCAGTTCCACATCACCGCGCTGTTCGAGCTGCCGCCTGATGGCGGAACCGACCAT
CCCGCGATGACCAGCAATAAAAACTCGTTGTTTACTCATTTTTTTCAAGCTTactagtGG
ATCCAGATCGGACGGGAAACGGTGCTTTCTGGTAGATATGGCCGCAACCGAAATCTTAAG
CAGTTACAGTGGTAGTAGTTGTGTCTGGCTTACGGTTGTAGTATGTTGTTAGGCCAATTT
ATTTAGGAAGTGGCTAGCTAACTTTATTGTTTGCTTAGATATATTTTTTCAATTATTTT
TCATTTTTTCATTTTTTTCATTTTTTTATTTTTTTATTTTTTTATTTTTTTATAATTGGC
TTCTTGTGGGATGTATAGTTTTTTTATTTTTCATACCGGATCTAATACTTGCAAACGAC
CGTACTTCAATCGTAGTGGTTCTATATGTGCTATATTTGAAATCGAAGATATTTCTGCCG
TTGCATCATCTAGCCTTTTCTCGATAAGATCTGCGGCCgcATCTACACGAAGAGGGAAAA
TTGACTCGTGCCAGGAAACTTGACCGTACCACTCTTTTCCATACCGTTAGAGATGTTTTA
CTTCATGATGGGTCTTACACCCGTCACAAAGGGGTCCGTTGTTTGGACAACGGTAGCGTT
AGAGTGAGCATACGGCTTAAGCACCGTCAATTAACCGCTACCTGGATAGAATTGGAACCC
ATTATTGAGAATGGCGAGGTGAAAGACGTTATGTTCAAATTGTCCACCAGAGTACAATAT
ACAGAGGAAAACGAGAACGAAACGCGCCCAGAATCTTCTTCAGAATGAGTGCATGTCAGA
CGCAGGCGTCGTATGCTTTTCATTAGTGATGCATGCCGTTGCAGCGTGCGAATCGGCACG
GTAATGATTCGAAATCTCTAATTATAATTACCTTCTGATATATAGATGAAAGACTATTTA
ATGAGAATATTGGACAGTACCGTTGGACTTCGCTGAGAGGTACATGGCATTTTGGTTAT
TGGATGTAGAATGGTTGAATAAACGTGATTGTAAAATAGAGTTTGTAACTACGAATAATT
AGTTTTTGAGAAGTTTGGTGAATTTAATATTTGTATGAGGAAAGTAAATTTTAATACCTA
AATAAACAAAAATATATGGTACAGGAACGCGAGGCAACGCGCCGATACAGGGTCAATGGG
TACACGAGAGGGTGACACTAGGCGTAGAAAGTCATTAGTATAAAATACAGTGGTATATAG
TAGATATTTAGTTTGTTTTCCTTTTCTTTTTCTCCAAACGATATCAGACATTTGTCTGAT
AATGAAGCATTATCAGACAAATGTCTGATATCGTTTTTCAATAATAATATACATCATCAC
AAAACAAACAAACATAGCATCGCAAGCCCCATCATGCCACCACCGTCCGCTGTGATCggc
gcgCCGCGGCTACTTTTCAATTCCCTATAGTGAGTCGTATTAAATTCGTAATCATGTCAT
AGCTGTTTCCTGTGTGAAATTGTTATCCGCTCACAATTCCACACAACATACGAGCCGGAA
GCATAAAGTGTAAAGCCTGGGGTGCCTAATGAGTGAGCTAACTCACATTAATTGCGTTGC
GCTCACTGCCCGCTTTCCAGTCGGGAAACCTGTCGTGCCAGCTGCATTAATGAATCGGCC
AACGCGCGGGGAGAGGCGGTTTGCGTATTGGGCGCTCTTCCGCTTCCTCGCTCACTGACT
CGCTGCGCTCGGTCGTTCGGCTGCGGCGAGCGGTATCAGCTCACTCAAAGGCGGTAATAC
GGTTATCCACAGAATCAGGGGATAACGCAGGAAAGAACATGTGAGCAAAAGGCCAGCAAA
AGGCCAGGAACCGTAAAAAGGCCGCGTTGCTGGCGTTTTTCCATAGGCTCCGCCCCCCTG
ACGAGCATCACAAAAATCGACGCTCAAGTCAGAGGTGGCGAAACCCGACAGGACTATAAA
GATACCAGGCGTTTCCCCCTGGAAGCTCCCTCGTGCGCTCTCCTGTTCCGACCCTGCCGC
TTACCGGATACCTGTCCGCCTTTCTCCCTTCGGGAAGCGTGGCGCTTTCTCATAGCTCAC
GCTGTAGGTATCTCAGTTCGGTGTAGGTCGTTCGCTCCAAGCTGGGCTGTGTGCACGAAC
CCCCCGTTCAGCCCGACCGCTGCGCCTTATCCGGTAACTATCGTCTTGAGTCCAACCCGG
TAAGACACGACTTATCGCCACTGGCAGCAGCCACTGGTAACAGGATTAGCAGAGCGAGGT
ATGTAGGCGGTGCTACAGAGTTCTTGAAGTGGTGGCCTAACTACGGCTACACTAGAAGGA
CAGTATTTGGTATCTGCGCTCTGCTGAAGCCAGTTACCTTCGGAAAAAGAGTTGGTAGCT
CTTGATCCGGCAAACAAACCACCGCTGGTAGCGGTGGTTTTTTTGTTTGCAAGCAGCAGA
TTACGCGCAGAAAAAAAGGATCTCAAGAAGATCCTTTGATCTTTTCTACGGGGTCTGACG
CTCAGTGGAACGAAAACTCACGTTAAGGGATTTTGGTCATGAGATTATCAAAAAGGATCT
TCACCTAGATCCTTTTAAATTAAAAATGAAGTTTTAAATCAATCTAAAGTATATATGAGT
AAACTTGGTCTGACAGTTACCAATGCTTAATCAGTGAGGCACCTATCTCAGCGATCTGTC
TATTTCGTTCATCCATAGTTGCCTGACTCCCCGTCGTGTAGATAACTACGATACGGGAGG
GCTTACCATCTGGCCCCAGTGCTGCAATGATACCGCGAGACCCACGCTCACCGGCTCCAG
ATTTATCAGCAATAAACCAGCCAGCCGGAAGGGCCGAGCGCAGAAGTGGTCCTGCAACTT
TATCCGCCTCCATCCAGTCTATTAATTGTTGCCGGGAAGCTAGAGTAAGTAGTTCGCCAG
TTAATAGTTTGCGCAACGTTGTTGCCATTGCTACAGGCATCGTGGTGTCACGCTCGTCGT

TTGGTATGGCTTCATTCAGCTCCGGTTCCCAACGATCAAGGCGAGTTACATGATCCCCCA
TGTTGTGCAAAAAAGCGGTTAGCTCCTTCGGTCCTCCGATCGTTGTCAGAAGTAAGTTGG
CCGCAGTGTTATCACTCATGGTTATGGCAGCACTGCATAATTCTCTTACTGTCATGCCAT
CCGTAAGATGCTTTTCTGTGACTGGTGAGTACTCAACCAAGTCATTCTGAGAATAGTGTA
TGCGGCGACCGAGTTGCTCTTGCCCGGCGTCAATACGGGATAATACCGCGCCACATAGCA
GAACTTTAAAAGTGCTCATCATTGGAAAACGTTCTTCGGGGCGAAAACTCTCAAGGATCT
TACCGCTGTTGAGATCCAGTTCGATGTAACCCACTCGTGCACCCAACTGATCTTCAGCAT
CTTTTACTTTCACCAGCGTTTCTGGGTGAGCAAAAACAGGAAGGCAAAATGCCGCAAAAA
AGGGAATAAGGGCGACACGGAAATGTTGAATACTCATACTCTTCCTTTTTCAATATTATT
GAAGCATTTATCAGGGTTATTGTCTCATGAGCGGATACATATTTGAATGTATTTAGAAAA
ATAAACAAATAGGGGTTCCGCGCACATTTCCCCGAAAAGTGCCACCTGACGCGCCCTGTA
GCGGCGCATTAAGCGCGGCGGGTGTGGTGGTTACGCGCAGCGTGACCGCTACACTTGCCA
GCGCCCTAGCGCCCGCTCCTTTCGCTTTCTTCCCTTCCTTTCTCGCCACGTTCGCCGGCT
TTCCCCGTCAAGCTCTAAATCGGGGGCTCCCTTTAGGGTTCCGATTTAGTGCTTTACGGC
ACCTCGACCCCAAAAAACTTGATTAGGGTGATGGTTCACGTAGTGGGCCATCGCCCTGAT
AGACGGTTTTTCGCCCTTTGACGTTGGAGTCCACGTTCTTTAATAGTGGACTCTTGTTCC
AAACTGGAACAACACTCAACCCTATCTCGGTCTATTCTTTTGATTTATAAGGGATTTTGC
CGATTTCGGCCTATTGGTTAAAAAATGAGCTGATTTAACAAAAATTTAACGCGAATTTTA
ACAAAATATTAACGCTTACAATTTCCATTCGCCATTCAGGCTGCGCAACTGTTGGGAAGG
GCGATCGGTGCGGGCCTCTTCGCTATTACGCCAGCTGGCGAAAGGGGGATGTGCTGCAAG
GCGATTAAGTTGGGTAACGCCAGGGTTTTCCCAGTCACGACGTTGTAAAACGACGGCCAG
TGCCAAGCTCCCGCGG (SEQ ID NO:22)

K.lactis s2 Promoter

GATCTTATCGAGAAAAGGCTAGATGATGCAACGGCAGAAATATCTTCGATTTCAAATATA
GCACATATAGAACCACTACGATTGAAGTACGGTCGTTTGCAAGTATTAGATCCGGTATGA
AAAATAAAAAAAACTATACATCCCACAAGAAGCCAATTATAAAAAAATAAAAAAATAAAA
AAATAAAAAAATGAAAAAAATGAAAAAATGAAAAATAATTGAAAAAATATATCTAAGCAA
ACAATAAAGTTAGCTAGCCCACTTCCTAAATAAATTGGCCTAACAACATACTACAACCGT
AAGCCAGACACAACTACTACCACTGTAACTGCTTAAGATTTCGGTTGCGGCCATATCTAC
CAGAAAGCACCGTTTCCCGTCCGATC (SEQ ID NO:23)

K.lactis s3 Promoter

GATCCTCTCTTTTTCCCCCTCATCGCATCTCATCTCGTCATAAACTTGACCATCATGTCA
ATCTTCTCAATATACACGAAAGAAGAAAAAAAAATTTTACATTGTAAAGAGGAAAAGC
TGTGAATCTTTTGAAATAAAATTTCAACCTTTAAGCCATTGAATTTTACCATTACGATAG
CACATTTTGCATAAAACAGGATAGAGGAAAAGAGGCACAGAGAGCTACTGCTGATC (SEQ ID NO:24)

A "purified protein" refers to a protein that has been separated from other proteins, lipids, and nucleic acids with which it is naturally associated. Preferably, the protein constitutes at least 10, 20, 50 70, 80, 90, 95, 99-100% by dry weight of the purified preparation.

An "isolated nucleic acid" is a nucleic acid, the structure of which is not identical to that of any naturally occurring nucleic acid, or to that of any fragment of a naturally occurring genomic nucleic acid spanning more than three separate genes. The term covers, for example: (a) a DNA which is part of a naturally occurring genomic DNA molecule, but is not flanked by both of the nucleic acid sequences that flank that part of the molecule in the genome of the organism in which it naturally occurs; (b) a nucleic acid incorporated into a vector or into the genomic DNA of a prokaryote or eukaryote in a manner, such that the resulting molecule is not identical to any naturally occurring vector or genomic DNA; (c) a separate molecule such as a cDNA, a genomic- fragment, a fragment produced by polymerase chain reaction (PCR), or a restriction fragment; and (d) a recombinant nucleotide sequence that is part of a hybridgene, i. e., a gene encoding a fusion protein. Isolated nucleic acid molecules according to the present disclosure further include molecules produced synthetically, as well as any nucleic acids that have been altered chemically and/or that have modified backbones.

Although the phrase "nucleic acid molecule" primarily refers to the physical nucleic acid molecule and the phrase "nucleic acid sequence" refers to the sequence of the nucleotides the nucleic acid molecule, the two phrases can be used interchangeably.

The term "substantially pure" in reference to a given polypeptide means that the polypeptide is substantially free from other biological macromolecules. The substantially pure polypeptide is at least 75% (*e.g.,* at least 80, 85, 95, or 99%) pure by dry weight Purity can be measured by any appropriate standard method, for example, by column chromatography, polyacrylamide gel electrophoresis, or HPLC analysis.

A "heterologous promoter", when operably linked to a nucleic acid sequence, refers to a promoter which is not naturally associated with the nucleic acid sequence.

The terms "express" and "over-express" are used to denote the fact that, in some cases, a cell useful in the method herein may inherently express some of the factor that it is to be genetically altered to produce, in which case the addition of the polynucleotide sequence results in over-expression of the factor. That is, more factor is expressed by the altered cell than would be, under the same conditions, by a wild type cell. Similarly, if the cell does not inherently express the factor that it is genetically altered to produce, the term used would be to merely "express" the factor since the wild type cell did not express the factor at all.

Engineered yeast cells displaying α(1,2) fucosylated glycans on the cell surface are potent pathogen adsorbents due to avidity effect (since the presentation these glycans are multivalent). The engineered yeast is provided as a food supplement - either as a live yeast or a killed (e.g. dried) yeast. The product is added to foods such as yogurts and kefir, or to weaning foods, or is included in a variety of other prepared foods, including cooked foods. In animal health applications the dried product, for example, is admixed with existing dried feedstuffs, e.g., in the form of kibble (e.g., for dogs) or pellets (e.g., for chickens, horses cattle). In the case of animal feedstuff, the yeast cells are combined with vegetable or meat (e.g., for dog food) or with corn and/or hay, soybeans, oats, wheat or other grains (e.g., for livestock).

Other features and advantages of the invention and disclosure will be apparent from the following description of the preferred embodiments thereof, and from the claims.

### BRIEF DESCRIPTION OF THE DRAWINGS

Figure 1 is a schematic illustration showing the synthetic pathway of the fucosyl oligosaccharides of human milk. *Se and Le* indicate synthesis by fucosyltransferases of the secretor and Lewis genes, respectively. The abbreviated biochemical name [with alternate biochemical structure in brackets] is given (histo-blood group antigen analog in parentheses).
Figure 2 is a photograph illustrating the effect of the expression of α(1,2) fucosyl glycans in Chinese hamster ovary (CHO) cells transfected with the FUT2 gene (α 1,2 FUT⁺) on the binding of *Campylobacter* (top right) and *V. cholerae* (below), compared to bacterial binding in parental CHO cells carrying the plasmid vector only (top left, α 1,2 FUT).
Figure 3 is a bar graph demonstrating the inhibition of CV387 capsid binding by human milk from mothers of various blood group phenotypes.
Figure 4 is a series of bar charts showing the concentration of fucosylated oligosaccharides in milk and the associated incidence of diarrhea in breastfed infants..
Figure 5 is an overall scheme for the generation of cell-surface α(1,2) fucosylated *K.lactis,* and includes examples of the recombinant genes necessary to achieve expression.
Figure 6 is a diagram illustrating the principle features of a recombinant DNA plasmid vector that, when transformed into *K.lactis,* directs the production of GDP-fucose in the cell.
Figure 7 is a diagram illustrating the principle features of a recombinant DNA plasmid vector that, when transformed into *K.lactis,* directs the production of GDP-fucose and 2'-fucosyllactose in the cell.
Figure 8 (Panel A) is a photograph of an immunoblot demonstrating expression of MYC-tagged *E.coli fcl,* MYC-tagged *E. coli gmd* and MYC-tagged *H.pylori futC in K.lactis* cells transformed with plasmid vectors containing the respective recombinant genes. Both single gene expression and combined two and three gene co-expression is demonstrated. (Panel B) is a photograph of a thin layer chromatogram of *K.lactis* cell extracts from cells co-expressing *futC, fcl* and *gmd* and grown in the presence of lactose. The TLC demonstrates the production of 2'-fucosyllactose.
Figure 9 is a scheme illustrating the pathways to GDP-mannose synthesis in yeasts.
Figure 10 is a diagram illustrating the principle features of recombinant DNA plasmid vectors that, when transformed into *K lactis,* direct the over-production of native or MYC-tagged versions of *K.lactis* phosphomannose isomerase (PMI, KLLA0D13728g) *K.lactis* phosphomannomutase (PMM, SEC53) or *K.lactis* GDP-mannose pyrophosphorylase (PSA1).
Figure 11 is a photograph of an immunoblot demonstrating over-expression of MYC-tagged *K.lactis* PMI, MYC-tagged *K.lactis-*PMM and MYC-tagged *K.lactis* PSA1 in *K.lactis* cells transformed with plasmid vectors containing the respective recombinant genes.
Figure 12 is a diagram (panel A) illustrating the *K.lactis* genome surrounding the GNT1 (MNN2-1) locus, encoding *N*-acetylglucosaminyltransferase. Also shown (panel B) is a diagram illustrating the principle features of pINT-1, a plasmid vector that does not replicate in *K.lactis* and that was designed to generate a specific 1000bp deletion in the GNT1 gene by homologous recombination.
Figure 13 is a diagram illustrating specific deletion using homologous recombination (in two steps) of most of the coding region (i.e. precisely 1000bp, starting at the initiator ATG codon) of *K.lactis* GNT1 (MNN2-1), encoding N-acetylglucosaminyltransferase.

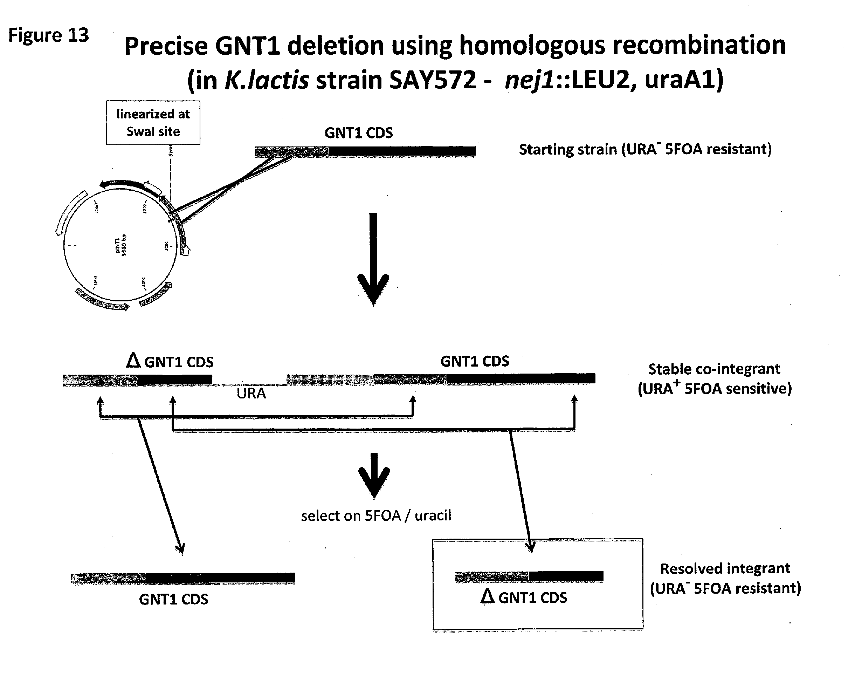

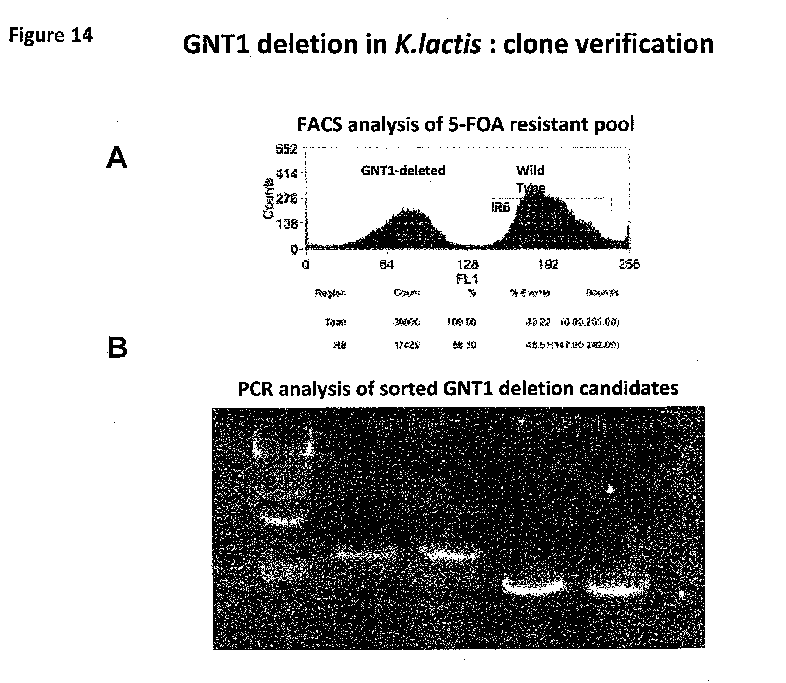

Figure 14 (panel A) is a plot of a FACS run performed on a pool of 5FOA resistant *K.lactis* cells (stained with FITC labeled *Griffonia simplicifonia* lectin II, GSII) that were spontaneously derived from a prior growth of URA⁺ SwaI-cleaved pINT-1 co-integrants (see Figure 13) under non-selective conditions. The low fluorescence peak on the left are cells which resolved the co-integrant by deleting GNT1, the high fluorescence peak on the right are cells which resolved the co-integrant by reverting back to wild-type. (panel B) is photograph of a stained agarose gel performed on PCR reactions using primers on either side of the specific deletion in GNT1. PCR products derived from the 5FOA resistant candidates are ∼ 1kb shorter than those derived from wild-type cells, consistent with the designed deletion of 1000bp.
Figure 15 is a photograph of an immunoblot demonstrating expression of MYC-tagged *E. coli fcl,* MYC-tagged *E.coli gmd,* MYC-tagged *H.pylori futC,* MYC-tagged *S.pombe* GMA12, MYC-tagged *S.pombe* PVG3, human FUT1 and human FUCT1 in *K.lactis* cells transformed with plasmid vectors containing the respective recombinant genes.

### DETAILED DESCRIPTION OF THE INVENTION AND DISCLOSURE

Adherence of pathogens to host cells is the obligatory first step in infection and is frequently mediated by specific molecular interactions. For example, *Campylobacter* species and Norwalk virus, the leading bacterial and viral causes of human infectious diarrhea, both adhere to gut epithelial surfaces through binding to specific sugars (*i.e*. α(1,2) fucosylated glycans) that are elaborated on the surface of intestinal cells. These same α(1,2) fucosylated glycans are also found in soluble forms in human breast milk, and recent work has demonstrated the critical role of these molecules, naturally provided by the mother to her baby, in the protection of infants from gut infections. For instance, there is a much higher risk of developing gut infections in infants: 1). at weaning, when protective factors found in mother's milk are reduced or removed from the child's diet, or 2). when infants are fed on conventional baby formula, which does not contain α(1,2) fucosylated glycans, or 3). for infants whose mothers are genetically incapable of providing sufficient α(1,2) fucosylated glycans in their milk.

The α(1,2) fucosylated glycans that are abundant in human breast milk effectively prevent binding of numerous important gut pathogens, both *in vitro* and *in vivo,* (such as Norwalk-like virus, or strains of bacteria from genera such as *Campylobacter* (e.g. *C. jejuni), Escherichia (e.g. E.coli), Salmonella (e.g. S. enterica) and Vibrio* (e.g. *V.cholerae)),* and thus prevent infection by these organisms. Thus, soluble α(1,2) fucosylated glycans present in human breast milk represent a class of natural anti-infective agents that provide an important layer of innate prophylactic protection to young infants. However, the production of α(1,2) fucosylated glycans as anti-infective agents in sufficient quantities to impact global diarrhea incidence remains a significant challenge. Chemical syntheses are possible, but are limited by stereo-specificity issues, product impurities, and high overall cost. *In vitro* enzymatic syntheses are also possible but are limited by a requirement for expensive nucleotide-sugar precursors. Accordingly, there is a pressing need for new strategies for α(1,2) fucosylated glycan-mediated inhibition of pathogen binding to gut epithelia.

### Travelers' diarrhea

Travelers' diarrhea is a very common illness affecting travelers. It is estimated that between 20-50% of international travelers develop the condition every year, representing an annual incidence of greater than 10 million cases. Although the disease is typically self-limiting (90% of cases resolve without treatment within one week), symptoms are unpleasant and disruptive, and international travelers, particularly to high-risk destinations such as the developing countries of Latin America, Africa, the Middle East and Asia, are advised to take dietary precautions while traveling to reduce their risk of contracting the illness. Moreover more than 10% of individuals who have recovered from travelers' diarrhea progress to inflammatory bowel syndrome (IBS), a chronic debilitating condition with no good current treatment options. Eighty percent (80%) of travelers' diarrhea cases are attributed to infections by enterotoxigenic *E. coli,* and are usually contracted by ingestion of fecally contaminated food or water. Many of the symptoms induced by enterotoxigenic *E. coli* are caused by small heat-stable peptide toxins secreted by the organism, and are mediated through toxin binding to an intestinal cell-surface receptor, the guanylin cyclase receptor. α(1,2) fucosylated milk-derived glycans inhibit this specific toxin binding and protect against enterotoxigenic *E*. *coli* infections.

Current treatments for travelers' diarrhea are mostly palliative, OTC formulations of gut anti-motility agents such as loperamide (Imodium®)) or diphenoxylate (Lomotil®) can provide symptomatic relief, but conversely can also delay pathogen clearance. Bismuth subsalicylate (Pepto-Bismol®) can be effective in alleviating symptoms, but is not an agent recommended for use by children under the age of 12, by pregnant women, or by people who are allergic to aspirin. Prophylaxis, other than by the unacceptable prophylactic use of antibiotics, is currently not an option against travelers' diarrhea. Diarrhea is a long-standing problem for the military and continues to be a dominant medical concern in deployed units. The compositions and methods described herein are useful in military-based applications. Probiotics

Probiotics are dietary supplements containing beneficial bacteria or yeasts. The World Health Organization characterizes probiotics as live microorganisms which when administered in adequate amounts confer a health benefit on the host.

The invention provides probiotic yeast strains expressing α(1,2) fucosylated glycans on their cell surface that act as "decoys", which adsorb pathogenic organisms on their cell surface with high efficiency (due to avidity effects). The strains are provided in foods or beverages such as yogurt, kefir, cultured drinks, or infant formula which are marketed as "probiotics". Alternatively, the microorganisms are provided dry, e.g., lyophilized, together with instructions for reconstitution by mixing with a liquid such as water, juice, or a dairy product.

The invention provides glycans representing a novel class of prophylactic and therapeutic agents. These agents are inexpensive to produce in large quantities, stable at room temperature, allow for large-scale storage, oral administration, and are safe and well tolerated. These glycans inhibit infection by a broad spectrum of pathogens, such as *Campylobacter jejuni,* caliciviruses (noroviruses, including Norwalk Virus), pathogenic vibrios, and certain diarrheagenic *Escherichia* and *Salmonella* strains (Chessa, D, Winter, MG, Jakomin, M and Bäumler, AJ, Mol Microbiol 71:4, 864-75 (2009)).

### Fucosyl glycans are receptors for pathogens

Attachment to the gastrointestinal mucosa is the essential first step in enteric infection. Cells express glycans on their surface to communicate with the external milieu, but these glycans are also used by pathogens for the process of host cell recognition and binding. Among cell surface glycans, those with fucosylated moieties are heavily expressed in intestinal mucosa and are used as target receptors for a number of enteric pathogens, including *Campylobacter* species, *Vibrio cholerae,* some diarrheagenic *E. coli,* human caliciviruses (noroviruses), *Helicobacter pylori,* and others (Ruiz-Palacios G. M. et al., 2003 J Biol Chem, 278:14112-14120; Marionneau S. et al., 2002 Gastroenterology, 122:1967-1977; Glass R.I. et al., 1985 Am J Epidemiol, 121:791-796; Barua D. and Paguio A.S., 1977 Ann Hum Biol, 4:489-492; Ilver D. et al., 1998 Science, 279:373-377; Newburg D.S. et al., 1990 J Infect Dis, 162:1075-1080; Huang P. et al., 2003 J Infect Dis, 188:19-31). For example, children who are homozygous recessive for the "secretor" α(1,2)-fucosyltransferase gene *(FUT2)* under-express α(1,2)-linked glycans on their intestinal mucosal surface and are naturally resistant to diarrhea; those with no such mutation in their *FUT2* alleles *(i.e.,* homozygous dominant for *FUT2)* express the highest level of these fucosylated receptors and have the highest risk of diarrheal disease. Thus, cell surface glycan receptors are critical determinants of pathogenicity for many enteric and other pathogens.

### Human milk glycans

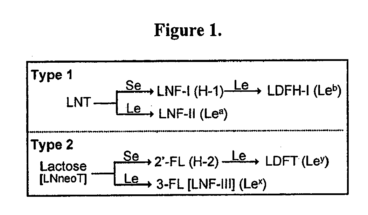

Human milk glycans, which comprise both unbound oligosaccharides and their glycoconjugates, play a significant role in the protection and development of the infant gastrointestinal (GI) tract. Milk oligosaccharides found in various mammals differ greatly, and composition in humans is unique (Hamosh M., 2001 Pediatr Clin North Am, 48:69-86; Newburg D.S., 2001 Adv Exp Med Biol, 501:3-10). Moreover, glycan levels in human milk change throughout lactation and also vary widely among individuals (Morrow A.L. et al., 2004 J Pediatr, 145:297-303; Chaturvedi P et al., 2001 Glycobiology, 11:365-372). Approximately 200 distinct human milk oligosaccharides have been identified and combinations of simple epitopes are responsible for this diversity (Newburg D.S., 1999 Curr Med Chem, 6:117-127; Ninonuevo M. et al., 2006 J Agric Food Chem, 54:7471-74801). Human milk oligosaccharides are composed of 5 monosaccharides: D-glucose (Glc), D-galactose (Gal), N-acetylglucosamine (GlcNAc), L-fucose (Fuc), and sialic acid (N-acetyl neuraminic acid, Neu5Ac, NANA). Human milk oligosaccharides are usually divided into two groups according to their chemical structures: neutral compounds containing Glc, Gal, GlcNAc, and Fuc, linked to a lactose (Galβ1-4Glc) core, and acidic compounds including the same sugars, and often the same core structures, plus NANA (Charlwood J. et al., 1999 Anal Biochem, 273:261-277; Martín-Sosa et al., 2003 J Dairy Sci, 86:52-59; Parkkinen J. and Finne J., 1987 Methods Enzymol, 138:289-300; Shen Z. et al., 2001 J Chromatogr A, 921:315-321). Approximately 70-80% of oligosaccharides in human milk are fucosylated. The majority of fucosylated oligosaccharides in most individuals contain one or more α(1,2)-linked fucoses, which are synthesized by a fucosyltransferase encoded by the *FUT2* gene. About one-third of human milk fucosyl oligosaccharides include one or more α(1,3) or α(1,4)-linked fucoses, synthesized by fucosyltransferases encoded by the Lewis gene *(FUT3)* family (Le Pendu, 2004 Adv Exp Med Biol, 554:135-143). Together, these fucosyltransferases produce six major epitopes (Figure 1; Type I and Type II pathways begin with different precursor molecules). In most mothers, the dominant milk oligosaccharides are 2'-fucosyllactose (2'-FL) and lacto-N-fucopentaose I (LNF-I). However, 20 to 25% of individuals of European, African and Asian, and 2-3% of Mexican ancestry are non-secretors, *i.e.,* they lack an active *FUT2* allele, and thus entirely lack α(1,2)-fucosyl glycans in their milk. These individuals have lower amounts of total oligosaccharide and a dominance of oligosaccharides containing only α(1,3) and α(1,4)-linked fucose.

### Human milk glycans inhibit binding of enteropathogens to their receptors

Human milk glycans have structural homology to cell receptors for enteropathogens and function as receptor decoys. For example, pathogenic strains of *Campylobacter* bind specifically to glycans containing H-2, *i.e.,* 2'-fucosyl-*N-*acetyllactosamine or 2'-fucosyllactose (2'FL); *Campylobacter* binding and infectivity are inhibited by. 2'FL and other glycans containing this H-2 epitope. Similarly, some diarrheagenic *E. coli* pathogens are strongly inhibited *in vivo* by human milk oligosaccharides containing 2-linked fucose moieties. Several major strains of human caliciviruses, especially the noroviruses, also bind to 2-linked fucosylated glycans, and this binding is inhibited by human milk 2-linked fucosylated glycans. Consumption of human milk that has high levels of these 2-linked fucosyloligosaccharides was associated with lower risk of norovirus, *Campylobacter,* ST of *E.* coli-associated diarrhea, and moderate-to-severe diarrhea of all causes in a Mexican cohort of breastfeeding children (Newburg D.S. et al., 2004 Glycobiology, 14:253-263; Newburg D.S. et al., 1998 Lancet, 351:1160-1164).

### Therapeutic glycans

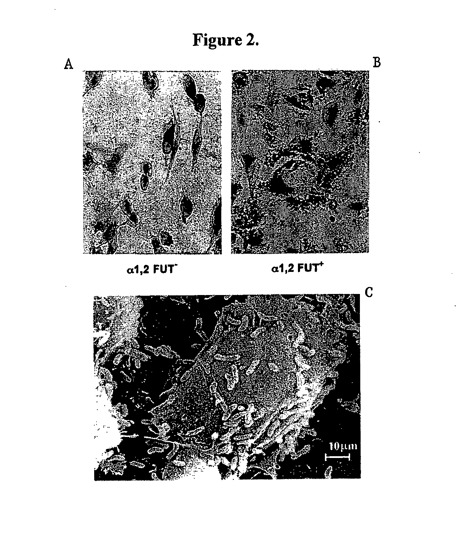

The human milk glycans represent a new class of powerful antimicrobial agents (Newburg D.S. et al., 2005 Annu Rev Nutr, 25:37-58; Sharon N. and Ofek I., 2000 Glycoconj J, 17:659-664), and the data indicate that they do not induce emergent drug-resistance. Glycan structures isolated from human milk were found to inhibit infection by specific pathogens *in vitro* and *in vivo.* Some pathogens *(e.g., C. jejuni, Vibrio cholerae)* are inhibited by a specific fucosylated epitopes in the monomeric form, *i.e.,* the free oligosaccharide, although inhibition is stronger with more complex forms. Other pathogens (e.g., noroviruses) are inhibited only by these epitopes when they are anchored to a macromolecule, as the polyvalent (multiple copies of the same epitope on a macromolecule) or multivalent (combinations of epitopes on a macromolecule) forms. In general, polyvalent and multivalent expression of oligosaccharides is accompanied by an increase in binding avidity to specific lectins. These observations indicate a need for multiple epitopes (as found in human milk) in a formulation that is intended to inhibit the multiplicity of pathogens found in a free-living population. However, 1) most human milk protective glycans contain in common only six neutral fucosylated glycan epitopes, the Lewis epitopes, which inhibit the most common major families of enteric pathogens (Figures 1 and 2) human milk contains only a few macromolecules that bind strongly to pathogens, and principal among them is the mucin expressed from the *MUC1* gene. For example, recombinant fucosylated mucin inhibits binding by *H. pylori.*

### Limitations of existing synthesis methods

While a wealth of published studies suggest that human milk glycans could be used as a novel class of antimicrobial anti-adhesion agents, the difficulty and expense of producing adequate quantities of these agents of a quality suitable for human consumption has limited their full-scale testing and perceived utility. Prior to the invention, there was a need for a suitable method for producing the appropriate glycans in sufficient quantities at reasonable cost. Synthetic approaches for glycan synthesis have been attempted. Novel chemical approaches can synthesize oligosaccharides, but reactants for these methods are expensive and potentially toxic. Enzymes expressed from engineered organisms provide a precise and efficient synthesis, but the high cost of the reactants, especially the sugar nucleotides, limits their utility for low-cost, large-scale production. Bacteria have been genetically engineered to express the glycosyltransferases needed to synthesize oligosaccharides from the bacteria's innate pool of nucleotide sugars. In one such example, all enzymes essential for oligosaccharide synthesis, including glycosyltransferases and the enzymes that comprise the nucleotide sugar regeneration pathway, were built into a single plasmid and expressed within one *E. coli* "superbug" (Chen X. et al., 2001 J Am Chem Soc, 123:8866-8867); oligosaccharides were synthesized on a g/L scale. However, bacteria produce cellular components, such as lipopolysaccharide and peptidoglycan, that are powerful antigens and toxins to humans thereby causing undesirable and dangerous side effects. Therefore, efforts were undertaken to develop cheaper and safer ways to manufacture human milk glycans.

### α(1,2) fucosylated glycans

α(1,2) fucosylated glycans present in human breast milk competitively inhibit the adherence of major pathogens to gut epithelia, and represent a class of anti-infective agents that provide prophylactic protection to young infants and adults against infection and/or colonization by gut pathogens. In addition to at risk infant populations, there exist several other additional large populations at risk for diarrheal disease (*e.g.* the elderly, international travelers, and military troops), who could benefit from prophylactic administration of these agents, were they readily available. However, as stated above, prior to the invention described herein, no current commercially feasible synthetic source of human breast milk-derived α(1,2) fucosylated glycans existed, and existing dairy products based on cow's or goat's milk are missing this class of molecules.

Prior to the invention, the production of α(1,2) fucosylated glycans as anti-infective agents in sufficient quantities to impact global diarrhea incidence was a significant challenge. Chemical syntheses are possible, but are limited by stereo-specificity issues, product impurities, and high overall cost (Flowers H.M., 1978 Methods Enzymol, 50:93-121; Seeberger P.H., 2003 Chem Commun (Camb), 1115-1121; Koeller K.M. and Wong C.H., 2000 Chem Rev, 100:4465-4494). *In vitro* enzymatic syntheses are also possible, but are limited by a requirement for expensive nucleotide-sugar precursors.

The invention describes less expensive ways of manufacturing these molecules in bulk as anti-infective agents. The invention also provides these agents as nutritional supplements and/or as therapeutics not only for at-risk infants (e.g., non-breast fed infants, partially breast-fed infants, breast-fed infants of mothers genetically incapable of providing sufficient protective glycans in their milk, weaning infants, infants in high risk environments for infectious diarrhea such as daycare centers, or locations with compromised water sanitation), but also for susceptible adults (e.g., the elderly, particularly in nursing home environments, for travelers, for all adults in high risk environments such as locations with compromised water sanitation). The invention also provides these agents as nutritional supplements or feedstuff additives for animals (e.g., pigs, cows, chickens) to reduce the risk of infectious diarrhea by organisms utilizing fucosylated glycans for pathogenesis (e.g., F18-fimbriated enterotoxigenic *E.coli* in pigs (Snoeck, V, Verdonck, F, Cox, E and Goddeeris, BM, Vet Microbiol 100:3-4, 241-6 (2004))

Glycan synthetic pathways were engineered in the common edible dairy yeast *Kluyveromyces lactis* through a combination of endogenous gene manipulation and the introduction of heterologous genes encoding desired activities. *K. lactis* was engineered to synthesize the key precursor sugar, GDP-fucose (optimizing the production of this nucleotide sugar and minimizing the impact of this on yeast cell wall synthesis by boosting the cellular GDP-mannose pool), the ability to transport both GDP-fucose and UDP-galactose to the Golgi, and the ability to elaborate α(1,2) fucosylated glycans on the cell surface. Yields of surface-bound α(1,2) fucosylated glycans were optimized. The engineered yeast was evaluated for pathogen binding in *vitro* and for use as a probiotic in an *in vivo* animal model of *Campylobacter* or *Vibrio* infection. By inhibiting pathogen adherence to epithelia, the engineered yeast is used to treat and/or prevent infection by various infections agents, *e.g.,* by inhibiting infectious agents associated with variou disorders such as enteric, pulmonary (airway), and urinary tract disorders. The surface α(1,2) fucosylated probiotic *K. lactis* targets both Norwalk-like viruses and C. *jejuni,* as well as other fucose-binding pathogens, including pathogenic *Vibrios, Helicobacter, Pseudomonas aerugionosa, Streptococcus pneumoniae,* and certain diarrheagenic *Escherichia* and *Salmonella* strains.

### Selection of expression host

*Kluyveromyces lactis* was selected to display α(1,2) fucosylated glycoproteins for a variety of reasons. First, the organism is eukaryotic and possesses intrinsic mechanisms for glycan production and secretion. The *K. lactis* genome has been completely sequenced, and the organism is benign and easily fermented on simple media. Moreover, since *K. lactis* is established in the human diet in various cheeses, appropriately engineered forms of the organism or products produced by the organism may qualify for GRAS status (Generally Recognized as Safe) with the Food and Drug Administration (Leclercq-Perlat M.N. et al., 2004 J Dairy Res, 71:346-354; Fadda, M. E. et al., 2001 Int J Food Microbiol, 69:153-156; Prillinger, H. et al., 1999 Antonie Van Leeuwenhoek, 75:267-283).

Although it is well established that the wide use of conventional anti-bacterial agents and antibiotics leads to the development of resistant strains, this problem is avoided with the fucosylated glycan adherence inhibitors (an advantage over conventional antibiotic approaches). Antibacterial agents typically act directly on the growth and/or viability of target organisms and impose severe selective pressures that provide opportunity for the development of resistance. Anti-adherence anti-infective agents do not exert this direct selective pressure, instead they render the pathogen's environmental niche unavailable. Thus the compositions described herein do not drive the development of resistance, because pathogens have not evolved mechanisms to circumvent the natural anti-infective glycans present in mother's milk.

### Compositions comprising engineered probiotic yeast

The engineered probiotic yeast, *e.g., K. lactis* that displays α(1,2) fucosylated glycans on its cell surface, are administered as an over-the-counter food, beverage, or nutraceutical product or as a pharmaceutical composition containing the engineered yeast and a pharmaceutically acceptable carrier, *e.g.,* phosphate buffered saline solution, mixtures of ethanol in water, water and emulsions such as an oil/water or water/oil emulsion, as well as various wetting agents or excipients. The engineered probiotic yeast are combined with materials that do not produce an adverse, allergic or otherwise unwanted reaction when administered to a patient. The carriers or mediums used include solvents, dispersants, coatings, absorption promoting agents, controlled release agents, and one or more inert excipients (which include starches, polyols, granulating agents, microcrystalline cellulose, diluents, lubricants, binders, disintegrating agents, and the like), etc. If desired, tablet dosages of the disclosed compositions are coated by standard aqueous ornonaqueous techniques.

The engineered probiotic yeast are administered orally, *e.g.,* as a tablet containing a predetermined amount of the probiotic yeast, pellet, gel, paste, syrup, bolus, electuary, slurry, capsule, powder, granules, as a solution or a suspension in an aqueous liquid or a nonaqueous liquid; as an oil-in-water liquid emulsion or a water-in-oil liquid emulsion, or in some other form. Orally administered compositions optionally include binders, lubricants, inert diluents, lubricating, surface active or dispersing agents, flavoring agents, and humectants. Orally administered formulations such as tablets may optionally be coated or scored and may be formulated so as to provide sustained, delayed or controlled release of the probiotic yeast. In another aspect, the agents of the invention are administered by rectal suppository, aerosol tube, naso-gastric tube, direct infusion into the GI tract or stomach or parenterally.

Pharmaceutical compositions containing probiotic yeast can also include therapeutic agents such as antiviral agents, antibiotics, probiotics, analgesics, and anti-inflammatory agents. The proper dosage is determined by one of ordinary skill in the art and depends upon such factors as, for example, the patient's immune status, body weight and age. In some cases, the dosage is at a concentration similar to that found for similar oligosaccharides present in human breast milk.

The probiotic yeast are added to other compositions. For example, they are added to an infant formula, a nutritional composition, a rehydration solution, a dietary maintenance or supplement for elderly individuals or immunocompromised individuals. The probiotic yeast is included in compositions that include macronutrients such as edible fats, carbohydrates and proteins. Edible fats include, for example, coconut oil, soy oil and monoglycerides and diglycerides. Carbohydrates include, for example, glucose, edible lactose and hydrolyzed cornstarch. Protein sources include, for example, protein source may be, for example, soy protein, whey, and skim milk. Compositions, including nutritional compositions, containing the probiotic yeast can also include vitamins and minerals (*e.g.,* calcium, phosphorus, potassium, sodium, chloride, magnesium, manganese, iron, copper, zinc, selenium, iodine, and Vitamins A, E, D, C, and B complex).

The engineered probiotic yeast displaying α(1,2) fucosylated glycans on the cell surface is delivered as a live culture, or alternatively as a preparation killed or substantially reduced in viability through treatments (e.g. drying) consistent with maintaining pathogen-binding activity for cell surface fucosylated glycans. Reduced viability forms of probiotic yeast are produced using know methods, e.g., flash-dried probiotic yeast is used as a convenient human food or animal feedstuff additive.

### Example 1. Heterogeneity of glycan expression in infants defines their risk of diarrhea

In children, the Lewis histo-blood group antigens in the gastrointestinal tract are thought to serve as primary receptors for several major enteropathogens. Genetic polymorphisms of fucosyltransferases in the infant underlie heterogeneous fucose expression in gut and other tissues, reflected as differences in expression of the Lewis blood group phenotype in erythrocytes and saliva, and especially as Lewis genotype, which is more precise. If binding to cell surface fucosylated glycans on the intestinal mucosa is the essential first step of pathogenesis, the risk of diarrheal infections in the infant should be related to heterogeneous expression of fucosylglycans (Marionneau S. et al., 2001 Biochimie, 83:565-573).

A cohort of 297 Mexican infants was tested and followed from birth to 2 yr of age (Noguera-Obenza M. et al., 2003 Emerg Infect Dis, 9:545-551). The relative risk of diarrhea varied by blood type (Table 1). Children expressing fucose primarily as the Fuc α(1,2) epitope (O, Lewis a-b-) had the highest risk of diarrhea, 3.0 cases/child-year. Those expressing fucose primarily as Fuc α(1,4) (O, Lewis a+b-), but lacking Fuc α(1,2) epitope, had the lowest risk, 1.5 cases/child-year (P=0.002). The A and B blood group types (all express the Fuc α(1,2) epitope, but with an additional sugar at the terminus) had intermediate risks of diarrhea; the extra sugars may sterically hinder access to the active epitope, and the production of A and B glycans may deplete the amount of glycans with the more active Fuc α(1,2) epitope.

**Table 1. Risk of diarrhea by Lewis ABO(H) blood type of humans**

| | | | | |
|---|---|---|---|---|
| **ABO(H) Lewis Group** | | | **All Diarrhea** | |
| | | **IR** | **RR (95%Cl)** | **P-Value** |
| O^{a- b-} (N=37) | 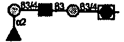 | 3.0 | 1.00 | |
| O^{a- b+} (N=191) | 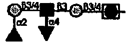 | 2.5 | 0.84 (0.72-0.99) | 0.04 |
| A^{a- b+} (N=55) | 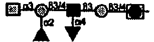 | 2.2 | 0.74 (0.61-0.91) | 0.003 |
| B^{a- b+} | 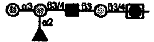 | | | |
| A^{a- b-} (N=7) | 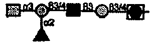 | 1.8 | 0.62 (0.29-0.79) | 0.001 |
| B^{a- b-} | 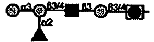 | | | |
| O^{a+ b-} (N=7) | 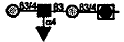 | 1.5 | 0.50 (0.40-0.92) | 0.01 |
| 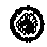 Gal ■GlcNAc ▲Fuco2 ▼Fucα4 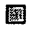 GalNAc | | | | |
| 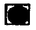 Residue could be glucose in the case of glyco lipids, or G lcNAc in the case of glycoprote Ins. | | | | |
| IR, Incidence Rates; RR. Relative Risk; Cl, Confidence Interv | | | | |

The differences in diarrhea susceptibility among blood group types indicate that human variation in glycan expression, specifically regarding expression of α(1,2)-linked fucose, is reflected in differences in pathogen susceptibility. Cell surface receptors containing α(1,2)-linked fucose are major determinants of pathogenicity of many enteric pathogens in human populations, because on the specificity of enteropathogen binding to their glycan receptors is an essential primary step in pathogenesis. Lewis blood group phenotypes and genotypes are powerful predictors of diarrhea in infants, with specific expression patterns rendering some children intrinsically more susceptible to these major enteropathogens.

### Example 2. Specific fucosyl glycans inhibit specific pathogens Glycan inhibition of Campylobacter

*Campylobacter jejuni* binding to HEp2 cells is inhibited by fucosylated carbohydrate moieties containing the H(O) blood group epitope (Fucα(1,2)Galβ(1,4)GlcNAc) (Ruiz-Palacios 2003). With nitrocellulose-immobilized neoglycoproteins (high-molecular-weight synthetic glycans), *Campylobacter* has high avidity for the H-2 antigen, and this specificity was confirmed by inhibition with monoclonal antibodies. *C. jejuni,* which normally does not bind to Chinese hamster ovary (CHO) cells, bound avidly when the cells were transfected with a human α(1,2)-fucosyltransferase gene that caused overexpression of H-2 antigen (Figure 2).

This binding was inhibited by ligands that bind to H-2, *e.g.,* UEA I, Lotus lectins and anti-H-2 mAbs, or by H-2 soluble mimetics, *e.g.,* H-2 neoglycoproteins, human milk fucosylated oligosaccharides and 2'FL, which compete with cell receptors. Thus, H-2 antigen was established as the host cell surface antigen that is the determinant of susceptibility to *Campylobacter* infection.

The relevance of inhibition of binding to the H-2 epitope in protection from disease was assessed in three models. Human milk oligosaccharides inhibited *Campylobacter* colonization in mice *in vivo* and inhibited invasive, pathogenic *Campylobacter* from binding to human intestinal mucosa ex *vivo.* Inhibition by 2-linked glycans was also assessed in transgenic animals. Female mice had been engineered to carry a human α(1,2)-fucosyltransferase gene (*FUT1*) expressed under the control of a whey acidic protein promoter that induces the expression of 2-linked fucose antigens in mammary gland during lactation, and thus, in milk. As a control, non-transgenic parental mice were used. Their suckling pups were challenged with inocula of *C. jejuni.* Up to 90% of non-transgenic controls remained colonized during follow-up. Colonization of transgenic mice was transient, and the time of colonization was directly related to the inoculum (Table 2). Thus α(1,2)-linked fucosylated glycoconjugates in milk protected against *Campylobacter* infection *in vivo,* confirming that the main intestinal ligands for *Campylobacter* are the H-2 histo-blood group antigens, and that milk fucosyloligosaccharides, specifically those containing α(1,2)-linked fucose, inhibit this binding.

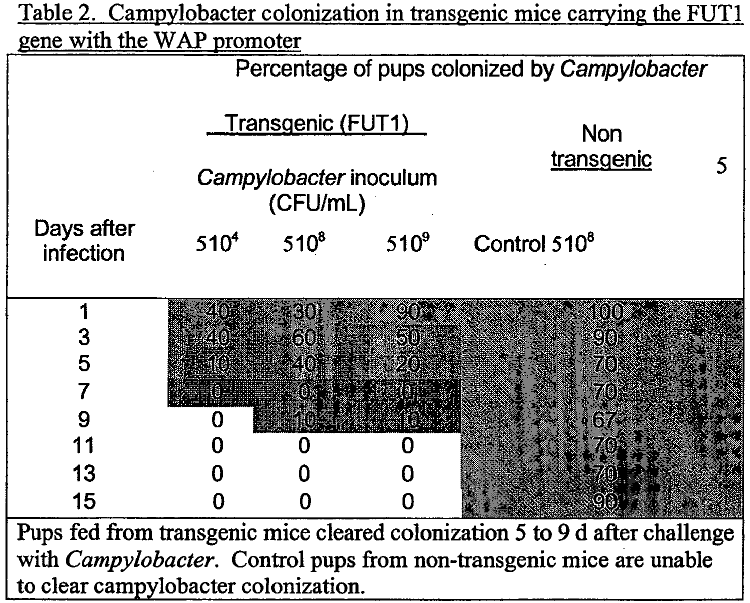

The human milk fucosyloligosaccharide fraction has greater inhibitory activity on a weight basis against *Campylobacter* than its major component, 2'FL.. The first step in searching for 2'FL homologs with greater inhibitory activity was to compare the activity of 2'FL, which contains a glucose on the reducing end, with 2'-fucosyl-*N*-acetyllactosamine (2'FLNAc), which is identical except that the sugar on the reducing end being N-acetylglucosamine. 2'FLNAc is the structural epitope found at the terminus of all higher homologs of 2'FL found naturally in human milk, including the macromolecular glycans. The 2'FLNAc is 18% more effective at inhibiting *Campylobacter* binding than 2'FL, consistent with the strongest inhibitors in human milk being the H-2 epitope expressed in macromolecules. Human milk contains a fucosylated macromolecular glycan that bound strongly to *Campylobacter.*

### Glycan inhibition of caliciviruses

The rabbit hemorrhagic disease virus (RHDV) specifically attaches to rabbit epithelial cells of the upper gastrointestinal and respiratory tracts through the H-2 epitope (Ruvoën-Clouet N. et al., 2000 J Virol, 74:11950-11954). VLPs of noroviruses (NVs) also bind to human gastro-duodenal epithelial cells derived from individuals of secretor phenotype, but not from non-secretor phenotype; non-secretors lack a functional α(1,2)-fucosyltransferase encoded by the *FUT2* gene, indicating that the α(1,2)-fucose epitope is essential for binding. The specificity of NV binding was determined by specific blocking of the binding by human milk from a secretor, by monoclonal antibodies specific for H-1 and H-3 antigens, by synthetic oligosaccharide conjugates containing secretor antigens, and by treatment of the tissues with α(1,2)-fucosidase. Transfection of CHO cells with α(1,2)-fucosyltransferase cDNA allowed attachment ofNV VLPs (Marionneau S. et al., 2002 Glycobiology, 12:851-856).

To determine the binding specificity of other strains of caliciviruses (CVs), saliva samples from 51 volunteers were tested for their ability to bind to eight recombinant capsid antigens representing seven genetic clusters of CVs (Huang 2003). The histo-blood group phenotypes were determined by monoclonal antibodies for Lewis and ABO antigens. Four patterns of binding were found: three of these (strains 387, NV, and MOH) bind secretors and one (strain 207) reacts with both non-secretors and secretors but prefers non-secretors. The three secretor-binding strains were further characterized based on the ABO antigen specificity: 387 recognizes all secretors (A, B, and O), NV recognizes A and O, and MOH recognizes A and B.

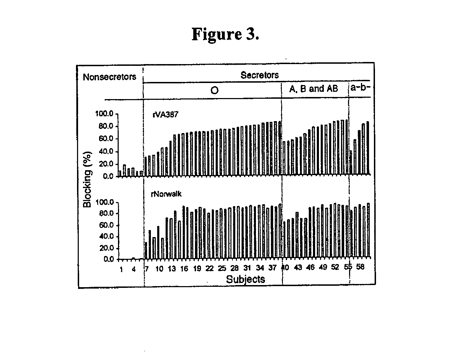

In 77 NV-challenged volunteers, 75% of the saliva samples from secretors, but none from the non-secretors, bound NV capsids (P<0.001). Secretors were almost 40 times more likely to become infected with NV than non-secretors, strongly suggesting that susceptibility to NV infection depends on secretor status. The ability of human milk to block binding of the most dominant strain of CV (VA387, genogroup II) to its receptor was tested (Figure 3). The milks from 54 of the 60 mothers blocked this common human enteropathogen; only the milks from the six non-secretors failed to block binding, indicating that the binding was inhibited by α(1,2)-linked fucose epitopes. A preparation of the free human milk oligosaccharides failed to inhibit binding, but the human milk high-molecular-weight glycan did bind to Norwalk virus, and did block binding of Norwalk virus to its receptor. The data indicate that human milk contains fucosylated glycans that specifically block CV binding to histo-blood group antigen receptors.

### Example 3. Fucosyl glycans in human milk inhibit diarrhea

Maternal Lewis histo-blood group type is associated with heterogeneity in expression of fucosylation patterns of oligosaccharides and fucosylglycans in milk. This innate variation in milk oligosaccharide expression was used to examine the effectiveness of naturally occurring human milk glycans to protect nursing infants against diarrhea.

Milk samples were obtained weeks 2-5 postpartum from 93 Mexican mothers; the mothers and infants were followed from birth up to 2 yr postpartum. The clinical histories of the infants were recorded prospectively, and concentrations of individual oligosaccharides in these milk samples were measured. The most common oligosaccharides in maternal milk were those that were fucosylated, comprising 73% (50%-92%) of total oligosaccharide. Of these, 2'FL, the oligosaccharide homolog of the Lewis H-2 epitope, was the most common in the milks of all mothers. The mean concentration of 2'FL in milk was 3854±108 nmol/mL (34% of total fucosylated oligosaccharides), but varied greatly among mothers according to their Lewis blood group type.

Breastfed infants who were infected with STEC (*i.e.,* exhibited stable toxin positive *E. coli* in their stools) were divided into two categories: Those exhibiting clinical symptoms of diarrhea (symptomatic), and those not exhibiting symptoms (asymptomatic). Those children who had symptoms of diarrhea were consuming milks with low relative amount of 2-linked oligosaccharides: the ratio of α(1,2)- to α(1,3/4)-linked fucosylated oligosaccharides in their mothers' milk was 3.9±0.7 [SE], (n=4), significantly lower than that of milks being consumed by infants who were also infected with STEC, but did not develop diarrhea (7.6±1.0, n=43), or uninfected controls (7.5±1.0, n=46) (P<0.01). Thus, higher concentrations of α(1,2)-linked oligosaccharides in milk (reflecting higher consumption by the infant) protects the infant against ST-associated diarrhea. This observation illustrates that variable expression of fucose epitopes in human milk is associated with differences in the ability of the milk to protect infants against a pathogen.

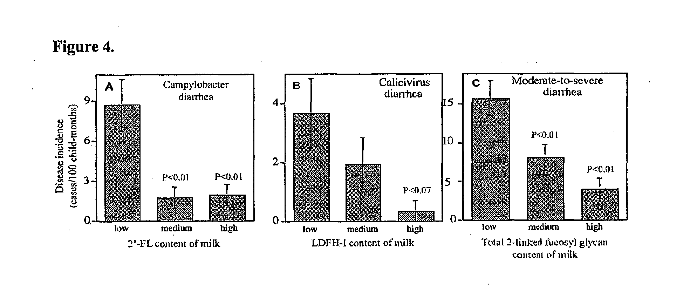

In these 93 nursing children consumption of milk containing high levels of 2'FL, but not of other oligosaccharides, was significantly associated with protection against *Campylobacter* diarrhea (Poisson regression, P=0.004; Figure 4 panel A). Consumption of milk containing high amounts of LDFH-I (another 2-linked fucosyloligosaccharide, which is the oligosaccharide homolog of the Le^{b} epitope), but not of the other oligosaccharides, was associated with protection against calicivirus diarrhea (Poisson regression, P=0.012; Figure 4 panel B). More generally, infants consuming milk relatively high in all measured 2-linked oligosaccharides were significantly (P<0.001) protected against diarrhea of all causes Figure 4 panel C). Thus, human milk containing high levels of specific oligosaccharides are associated with decreased risk of pathogen-specific diarrhea and diarrhea overall. For some pathogens, these milk oligosaccharides *per se,* as well as high-molecular-weight milk components containing these specific glycan epitopes, inhibit the pathogens *in vivo* and in *vitro;* in others, only the high-molecular-weight components in milk that contain the relevant epitope exhibit inhibition. Because both the oligosaccharide and the high-molecular-weight glycans that share the same glycan epitope are synthesized by the same fucosyltransferase, the relationship between the oligosaccharide expression in milk and decreased risk in the infant reflects the synthetic capacity of the mother for both the free oligosaccharide and the homologous glycan. Specific human milk oligosaccharides protect breastfed children from disease by inhibiting binding of pathogens to the child's intestinal receptors.

### Example 4. GDP-fucose synthesis in the K. lactis cytoplasm

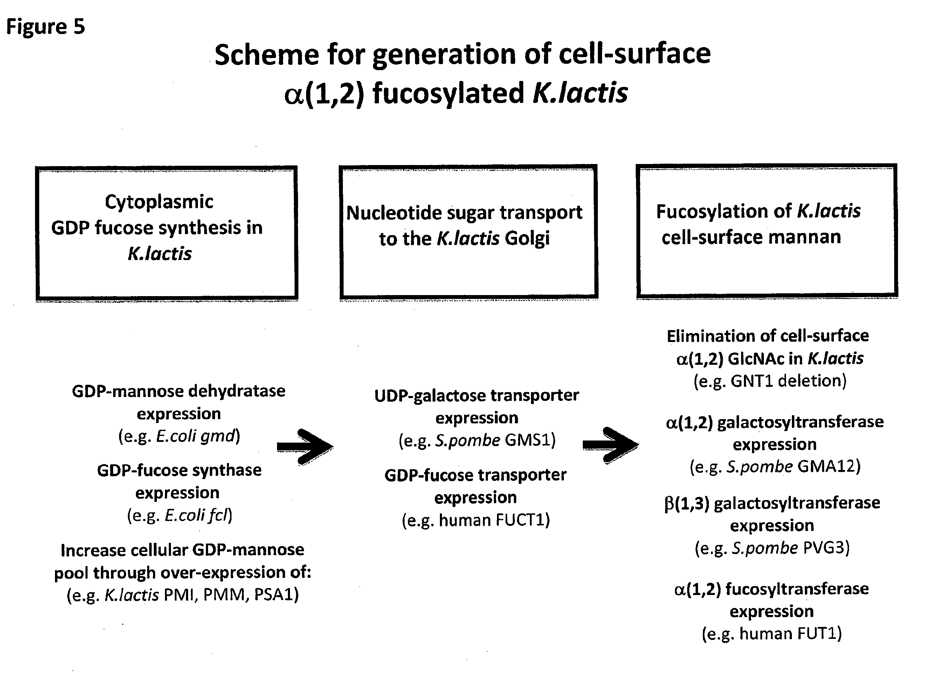

The yeast *Kluyveromyces lactis,* already a common constituent of human foods such as cheeses, is known to survive well in the conditions found in the human alimentary tract. *Kluyveromyces lactis* cells, modified through the steps outlined below (and summarized in Figure 5) to display α(1,2) fucosylated glycans on their cell surface, when taken orally either as specific preparations, or as constituents of foods and beverages, act as high avidity decoys within the gut, adsorbing pathogens and providing resistance to infection. *Kluyveromyces lactis* is modified as follows to provide it with a new ability to display α(1,2) fucosylated glycans on its cell surface.

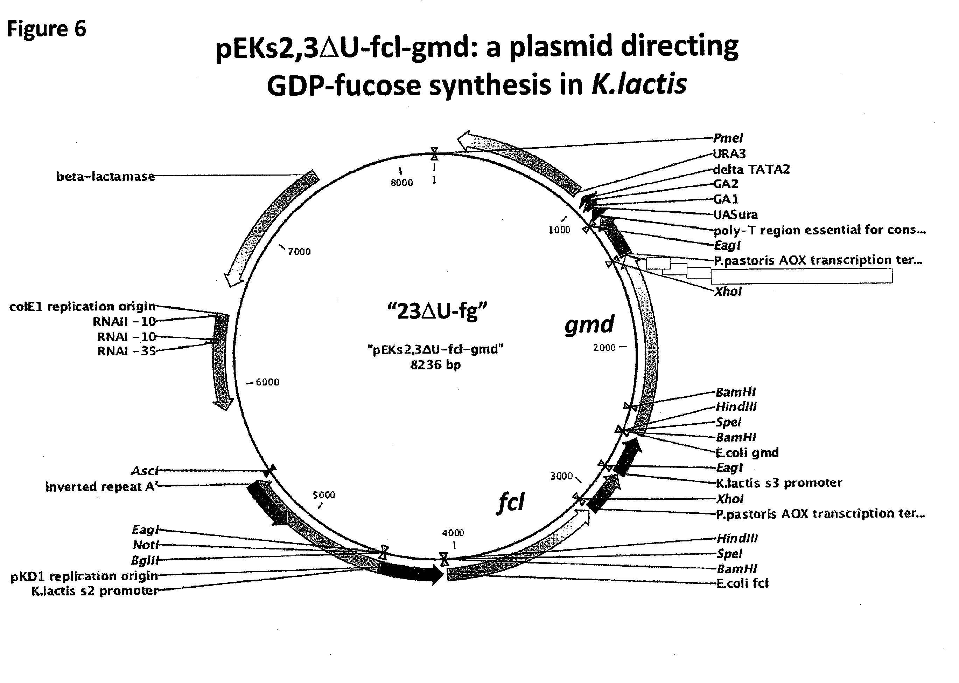

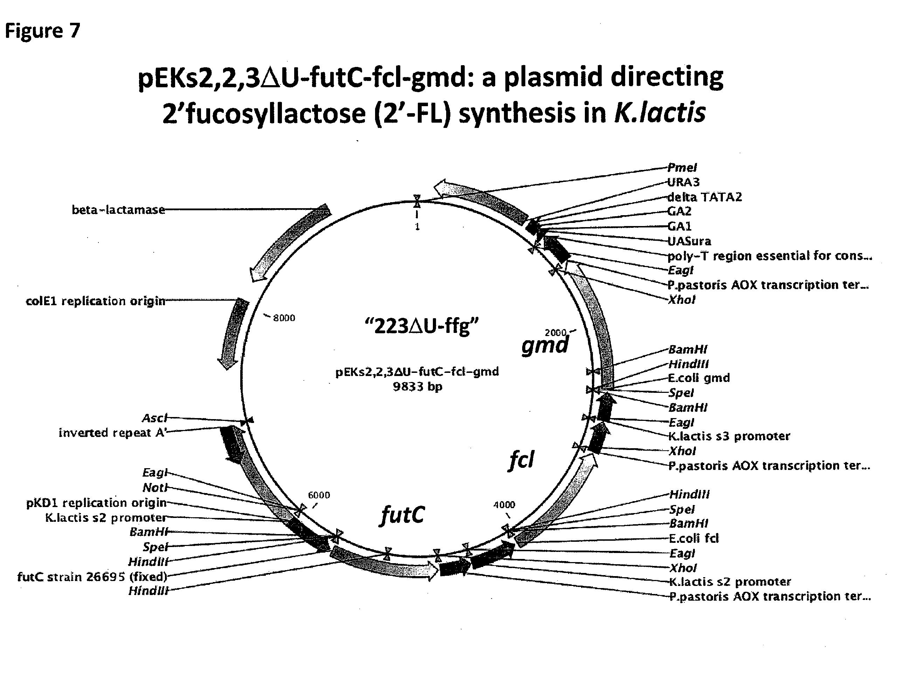

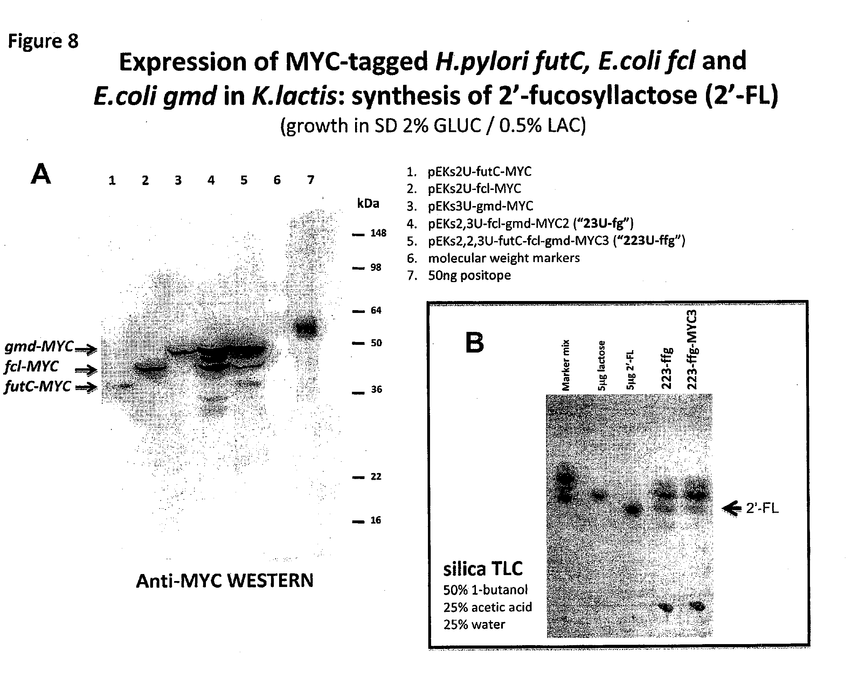

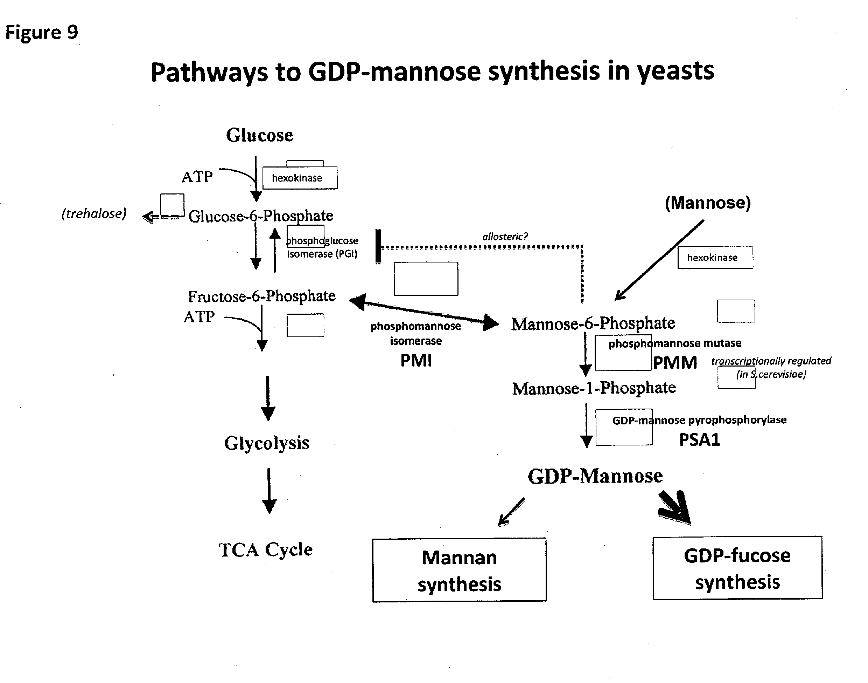

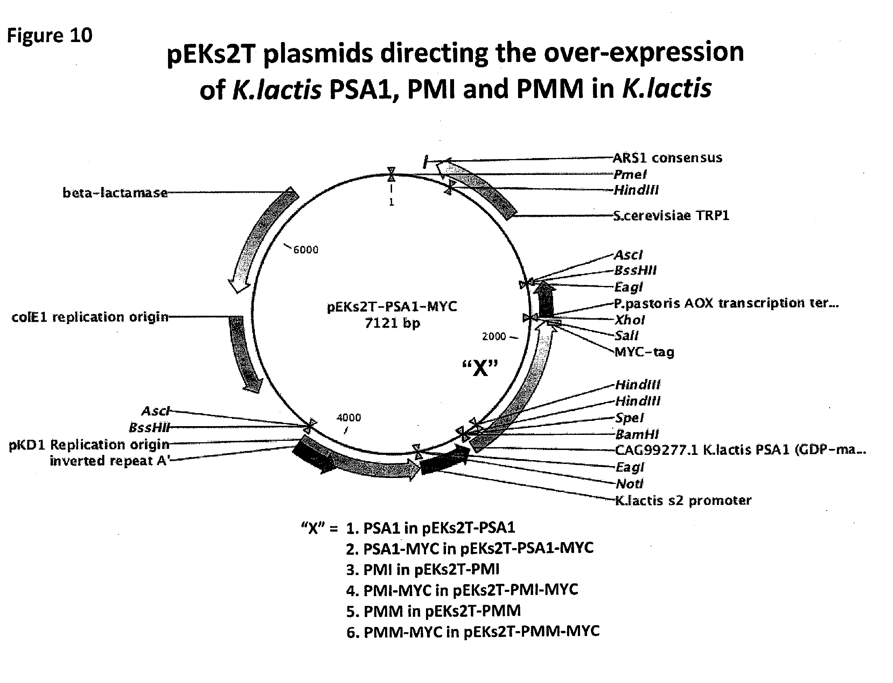

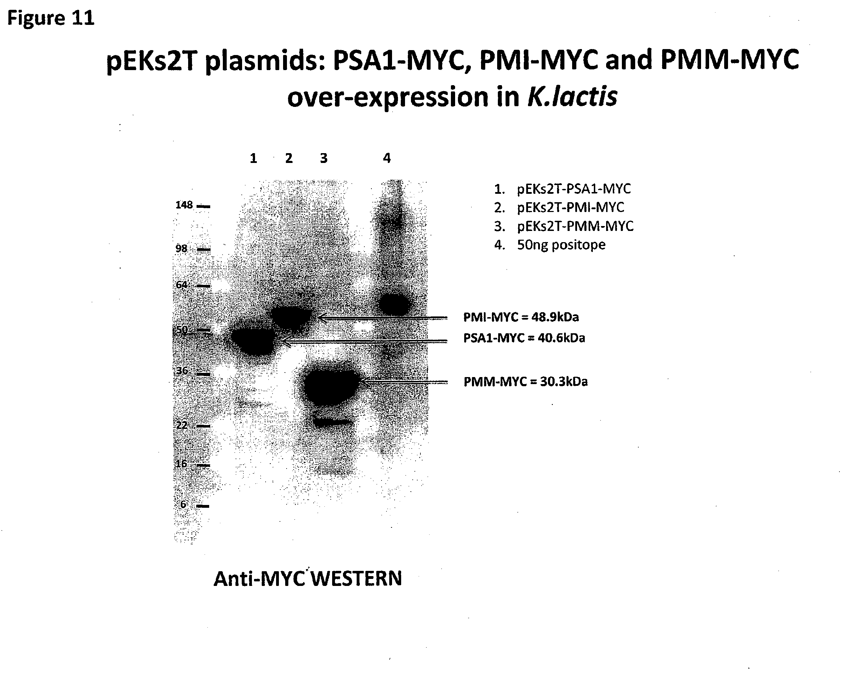

*Kluyveromyces lactis* lacks the ability to make GDP-fucose, a key precursor for synthesis of fucosylated glycans. Thus, an essential first step is to provide *K. lactis* with the means to generate this nucleotide-sugar. The *K. lactis* cell wall comprises glycoproteins (mannoproteins) that carry covalently linked mannose and glucose/glucosamine polymers (Gemmill T.R. and Trimble R.B., 1999 Biochim Biophys Acta, 1426:227-237). In general, yeast mannan synthesis requires an abundant supply of precursors; in particular a large amount of GDP-mannose is produced in the yeast cell cytoplasm for transport into the Golgi where it is used in mannosylation reactions. Mutations eliminating or materially reducing GDP-mannose synthesis or mannosylation in yeasts are lethal or deleterious. In the surface-engineered strains of the current invention a portion of cellular GDP-mannose flux is diverted toward the production of cytoplasmic GDP-fucose, while overall cell viability is maintained. GDP-mannose is converted to GDP-fucose in a three-step reaction catalyzed by two enzymes; GDP-D-mannose dehydratase (GMD) and GDP-L-fucose synthetase (GFS). Genes encoding these enzymes have been isolated from a number of organisms, including *E. coli, Helicobacter pylori* (Wu B. et al., 2001 Biochem Biophys Res Commun, 285:364-371) and humans (Sullivan F.X. et al., 1998 J Biol Chem, 273:8193-8202). These genes were introduced into *K. lactis* (strain "K25": MATa, uraA trp1 lysA1 lac4-8 [pKD1+]) either positioned on plasmids, such as the plasmid pEKs2,3ΔU-fcl-gmd (Figure 6, SEQ ID:1), or as stable chromosomal insertions. pEKs2,3ΔU-fcl-gmd is a shuttle vector capable of stable replication in *E. coli* (by virtue of a pUC-18 origin of replication and an ampicillin resistance selectable marker gene), or alternatively in *K.lactis* (by virtue of a pKD-1 origin of replication, allowing replication in pKD-1 ⁺ strains, and a *S.cerevisiae* URA3 selectable marker gene). pEKs2,3ΔU-fcl-gmd carries two strong *K.lactis* promoters, s2 and s3, which constitutively drive the expression of *E. coli fcl* and *gmd* genes, respectively. Downstream of both *fcl* and *gmd* in pEKs2,3ΔU-fcl-gmd are positioned copies of a transcription terminator based on that of the alcohol oxidase gene of *P.pastoris* (AOXter). A linear ∼4.5kb DNA fragment carrying s2-*fcl*, *s3-gmd,* and *S.cerevisiae* URA3 can be conveniently prepared from pEKs2,3ΔU-fcl-gmd by digestion with NotI and PmeI. In some embodiments of the current invention, this fragment was transformed into *K.lactis* by electroporation and efficiently incorporated into genomic DNA through non-homologous recombination, a mechanism that is extremely active in this organism. *E. coli* GMD and GFS enzymes have previously been introduced into the yeast *Saccharomyces cerevisiae* and shown to direct the synthesis of GDP-fucose (Mattila P. et al., 2000 Glycobiology, 10:1041-1047). Several assays to determine GDP-fucose levels are known in the art. To demonstrate GDP-fucose production in engineered *K.lactis,* we chose to combine enzymatically GDP-fucose was combined with lactose in the *K.lactis* cytoplasm to generate 2'-fucosyllactose (2'-FL), the presence of which was conveniently assayed by thin layer chromatography (TLC). Figure 7 shows pEKs2,2,3ΔU-futC-fcl-gmd, a plasmid based on pEKs2,3ΔU-fcl-gmd, but with an additional s2-futC-AOXter expression cassette inserted 5'- of *fcl.* (*futC* is an α(1,2) fucosyl transferase gene from *Helicobacter pylori).* Figure 8, panel A, shows individual expression of MYC-tagged *futC, fcl* and *gmd* (lanes 1-3) directed by plasmids based on "pEKs2,3ΔU-fcl-gmd" but that carry only single gene cassettes. Co-expression in the same cell of *fcl* and *gmd,* directed by pEKs2,3ΔU-fcl-gmd, is shown in lane 4. Co-expression of *fcl, gmd* and *futC* in the same cell, directed by pEKs2,2,3ΔU-futC-fcl-gmd, is shown in lane 5. Figure 8, panel B, shows the production of 2'-FL in cells co-expressing *fcl, gmd* and *futC* and grown in the presence of lactose. The production of 2'-FL demonstrates that GDP-fucose was successfully made from GDP-mannose in *K.lactis.* To mitigate the impact on cell viability due to depletion of cellular GDP-mannose, the cellular GDP-mannose pool was enhanced in *K.lactis* through over-expression of the *K.lactis* genes encoding three essential enzymes in the flow of carbon to GDP-mannose from central glucose metabolism: phosphomannose isomerase (PMI, KLLA0D13728g), phosphomannose mutase (PMM, SEC53) and GDP-mannose pyrophosphorylase (PSA1, (Uccelletti D. et al., 2005 FEMS Yeast Res, 5:735-746)). Figure 9 shows the pathway of GDP-mannose synthesis in yeasts, and the positioning of these three enzymes in this scheme. External mannose in the growth medium can also directly enter into this pathway, as shown in Figure 9, and mannose inclusion in growth media is an option in some embodiments. Figure 10 shows pEKs2T-PSA1-MYC, a plasmid based on pEKs2,3ΔU-fcl-gmd, but with a single s2-PSA1-MYC cassette, and with the *S.cerevisiae* URA3 gene replaced by *S.cerevisiae* TRP1. In *K.lactis* strain "K25" TRP1- and URA3-carrying plasmids can replicate and co-exist in the same cell in dual selective media. pEKs2T-based vectors were constructed for MYC-tagged and untagged versions of PMI, PMM and PSA. A single . pEKs2T-based vector was also constructed that made all three enzymes in the same cell simultaneously, and at the same time as *fcl* and *gmd* were expressed by pEKs2,3ΔU-fcl-gmd. Figure 11 shows robust over-expression of MYC-tagged PMI, PMM and PSA1 utilizing pEKs2T-based plasmid constructs.

The cytoplasmic pool of GDP-mannose may be elevated in certain non-lethal mutants of the yeast GDP-mannose Golgi transporter, VRG4 (Gao X.D., 2001 J Biol Chem, 276:4424-4432). Use of these mutations also allows for increased production of GDP-fucose GMD/GFS (*gmd*/*fcl*) are sensitive to feedback inhibition, which may limit the overall yield of cytoplasmic GDP-fucose achieved. However, provision of a GDP-fucose "sink" relieves this inhibition (*e.g*., through providing an enzyme in the cell cytoplasm to consume GDP-fucose in generating a fucosylated product, or a transporter to move the GDP-fucose into a different cellular compartment (see below)).

Long-term stability of engineered strains is important in commercial manufacturing, so consideration is given to chromosomal versus episomal location of the introduced recombinant genes, and to the choices of selectable markers for episomal maintenance.

Species origin for the GMD and GFS introduced into *K. lactis* is selected by GRAS considerations, *i.e*., genes derived from non-pathogens is preferred.

### Example 5. Transport of cytoplasmic GDP-fucose and UDP-galactose into the K. lactis Golgi

In eukaryotes, glycosylation of secreted proteins occurs in the ER and Golgi, where membrane-bound glycosyltransferases utilize luminal GDP-, UDP- or CMP- sugar substrates (nucleotide-sugars) to glycosylate suitable protein acceptors. The various nucleotide-sugars are exported to the lumen of the ER and Golgi from the cell cytoplasm by transmembrane nucleotide-sugar transporter proteins (NSTs). NSTs can be monospecific, transporting only one particular nucleotide-sugar, or di- or multi-specific, exhibiting relaxed substrate specificity. Particular organisms may entirely lack the ability to transport particular nucleotide-sugars, depending on the types of glycan they have evolved to elaborate.

All human H- (and Lewis) antigens contain both a fucose and a galactose moiety. Steps to engineer cytoplasmic synthesis of GDP-fucose *in K. lactis* were described in example 4, and cytoplasmic UDP-galactose is a normal *K. lactis* metabolite. However, *K*. *lactis* has not been shown to possess intrinsic abilities to import either GDP-fucose or UDP-galactose into the Golgi. The invention provides for the development of a strain of *K. lactis* that is competent for the Golgi import of both GDP-fucose and UDP-galactose.

UDP-galactose transport to the golgi has not been described for wild type *K.lactis.* The complete genome sequence ws found to include the presence of a putative gene (locus Q6CR04, Homolog of UDP-galactose Transporter (HUT1)) that possesses significant homology to other characterized UDP-galactose transporters. Moreover, *Saccharomyces cerevisiae* contains a homologous gene (HUT1), 53% identical at the amino acid level to *K*. *lactis* HUT1, which has been experimentally linked to an increased ability to incorporate galactose into glycans (in the presence of a heterologous galactosyltransferase) (Kainuma M. et al., 2001 Yeast, 18:533-541). The innate ability of *K. lactis* to transport UDP-galactose to the Golgi can be readily checked utilizing radiolabelled UDP-galactose and *K.lactis* golgi membrane vesicles prepared using published procedures (Gao, XD, Nishikawa, A and Dean, N, J Biol Chem 276:6, 4424-32 (2001)). Additional UDP-galactose transporter activity is provided to *K.lactis* by over-expression of a heterologous UDP-galactose transporter gene using an expression cassette, vector and strain as outlined in example 4. Examples of heterologous UDP-galactose transporters are used are the human UDP-galactose transporter (UGT1) (Miura N.I et al., 1996 J Biochem, 120:236-241), the *S.pombe* UDP-galactose transporter (GMS1) (Kainuma, M, Ishida, N, Yoko-o, T, Yoshioka, S, Takeuchi, M, Kawakita, M and Jigami, Y , Glycobiology 9:2, 133-41 (1999)), or the *S.cerevisia* UDP-galactose transporter (HUT1) described above.

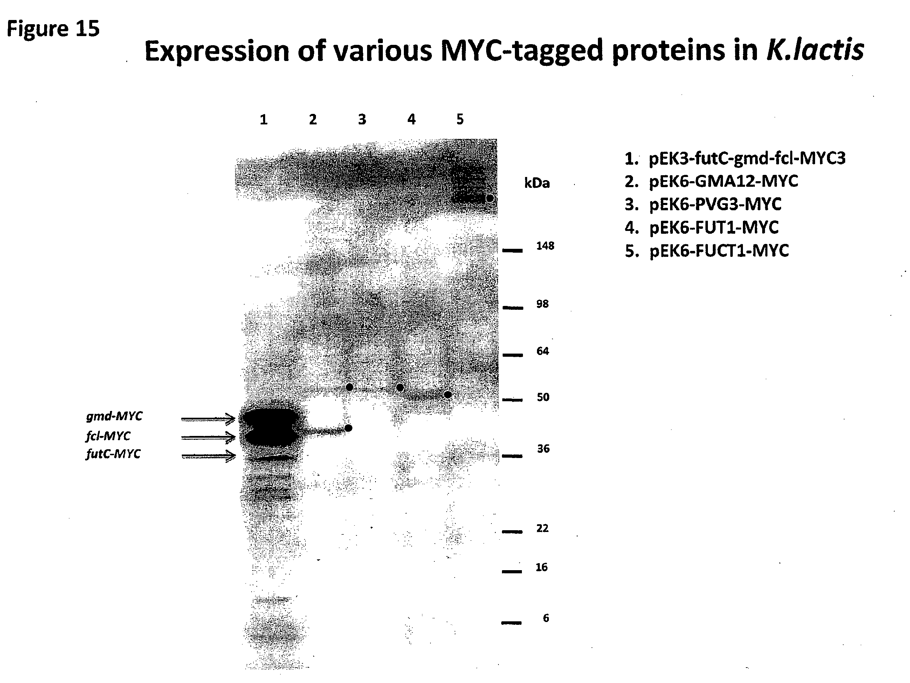

Golgi import of GDP-fucose in humans is achieved through a fucose-specific NST, FUCT1, mutations in which are responsible for leukocyte adhesion deficiency II. The direct approach to ensure Golgi transport of GDP-fucose in *K. lactis* is to express human FUCT1 in the organism. Figure 15, lane 5 demonstrates expression of MYC-tagged human FUCT1 in *K.lactis,* utilizing using an expression cassette, vector and strain as outlined in example 4. A broad range of known GDP-fucose transporters are optionally expressed in *K.lactis* in order to transport GDP-fucose into the golgi, (e.g., GDP-fucose transporters from other mammalian species such as dogs, mice, pigs and cows, or from birds (e.g. chickens) or fish (e.g. zebrafish), or many other species. An alternative approach is the introduction into *K.lactis* of a multi-specific NST, such as the *Leishmania* LPG2 protein (Segawa H. et al., 2005 J Biol Chem, 280:2028-2035). Since LPG2 has the ability to transport both GDP-mannose and GDP-fucose, it is a useful tool in balancing the relative synthesis and transport of these two nucleotide sugars. Controlling this balance is important for maintaining strain viability while maximizing overall fucosylated product yield. One immediate indicator of success for Golgi GDP-fucose transport in the engineered strain is increased total cellular GDP-fucose levels through relief of product feedback inhibition on cytoplasmic GDP-L-fucose synthetase. A more direct indication of functional activity of human FUCT1, or *Leishmania* LPG2, or human UGT1 in *K. lactis* is measurements of labeled nucleotide-sugar uptake into purified yeast Golgi vesicle preparations (Gao, XD, Nishikawa, A and Dean, N, J Biol Chem 276:6, 4424-32 (2001)).

### Example 6. Elimination of α(1,2) GlcNAc in K. lactis mannans

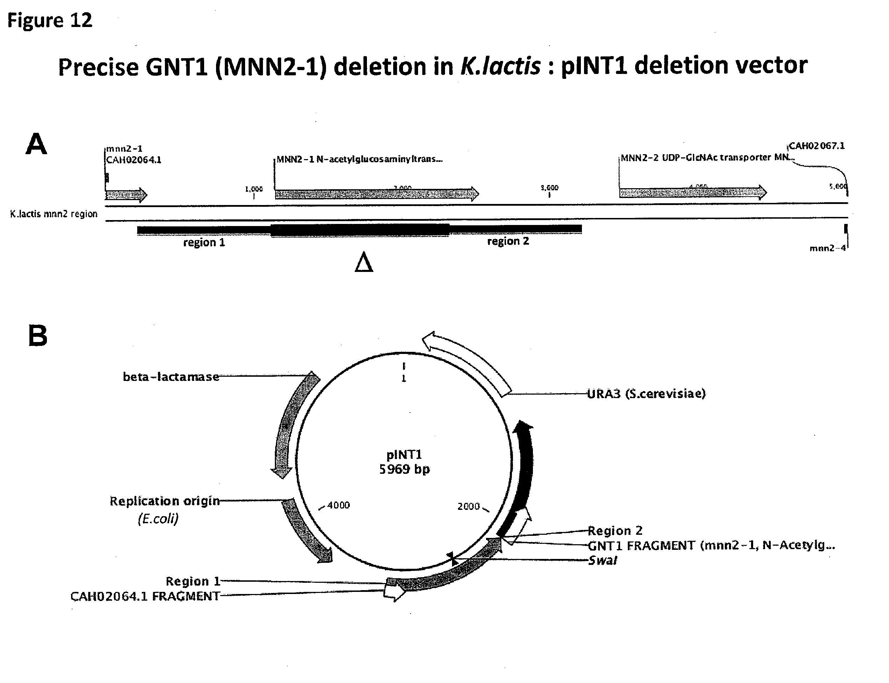

The mannans of the *K. lactis* cell wall comprise linear α(1,6)-linked mannose backbones to which are attached branched α(1,2)-linked mannose side chains. Many of these branches are terminated by a single α(1,3)-mannose, with the penultimate α(1,2) mannose group at termini frequently being modified with α(1,2)-linked N-acetylglucosamine (Guillen E. et al., 1999 J Biol Chem, 274:6641-6646). This α(1,2) GlcNAc is not a suitable acceptor for galactose addition, and thus it is desirable to eliminate GlcNAc from *K*. *lactis* mannan to clear the way for galactose and fucose additions. The formation of terminal α(1,2) GlcNAc is prevented by the introduction of either one of two previously described *K. lactis* mutations, *mnn2-1* and *mnn2-2* (Smith W. L. et al., 1975 J Biol Chem, 250:3426-3435). The former abolishes N-acetylglucosaminyltransferase activity, the latter removes UDP-GlcNAc Golgi transport activity. *K. lactis* strains carrying either mutation are viable, while the presence of either results in mannan lacking terminal a(1,2)-GlcNAc. Figure 12, panel A illustrates the *K.lactis* genome surrounding the GNT1 (MNN2-1) locus, encoding N-acetylglucosaminyltransferase. Approximately 3kb of *K.lactis* chromosomal DNA, including the entire GNT1 (MNN2-1) gene and ∼1kb of 5' and 3' flanking sequences, was cloned into an *E.coli* plasmid. A precise 1000bp DNA segment designated "Δ" in the figure, whose 5'-end was the GNT1 methionine initiator codon, was then deleted to generate the integration vector pINT-1 (Figure 12, panel B). This vector, which is was not capable of replication in *K.lactis,* was linearized with restriction endonuclease SwaI as indicated, and transformed by electroporation into *K.lactis* strain SAY572 (uraA1 trp1 leu2 lysA1 metAl nej::LEU2) (Kegel, A, Martinez, P, Carter, SD and Aström, SU. Nucleic Acids Res 34:5, 1633-45 (2006)). SAY572 is deficient in non-homologous recombination, and URA⁺ colonies derived from the transformation survived by incorporating the linear pINT-1 by homologous recombination at the GNT1 locus, generating a cointegrant structure as outlined in Figure 13. Following an outgrowth on non-selective medium, resolved, URA⁻, cointegrants were isolated by plating onto medium containing 5FOA and uracil. Figure 14, panel A, shows a FACS scan of a pool of recovered URA⁻ clones (stained with fluorescently labeled *Griffonia simplicifonia* lectin II, GSII, specific for GlcNAc). The two observed peaks were as expected, one (FACS bright) represented clones that had resolved back to wild type, the second peak (FACS dark) were clones that had resolved to maintain the 1000bp GNT1 deletion. Figure 14, panel B shows confirmation of this deletion by PCR in two clones purified from the FACS dark population. These clones do not incorporate GlcNAc into their mannan.

### Example 7. Addition of α(1,2) galactose residues onto K. lactis surface polymannose

A galactose molecule is added to the *K. lactis* surface polymannose (in the GNT1 null mutant background of example 6) to serve as a foundation for subsequent galactose and fucose additions. *Schizosaccharomyces pombe* possesses an α(1,2) galactosyltransferase, the product of the *gma12* gene that is able to link galactose to mannose acceptors (Chappell T.G., et al., 1994 Mol Biol Cell, 5:519-528). GMA12 enzyme incorporates galactose into cell wall mannans in *S. cerevisiae* (Kainuma M. et al., 1999 Glycobiology, 9:133-141). Figure 15, lane 2, demonstrates expression of MYC-tagged *S.pombe* GMA12 in *K.lactis,* utilizing using an expression cassette, vector and strain as outlined in example 4. Galactose incorporation by GMA12 is more efficient in a *mnn1* strain background (*i.e.*, deficient in α (1,3) mannosyltransferase) (Kainuma M. et al., 1999 Glycobiology, 9:133-141). *K. lactis mnn1* mutants are employed here to optimize the yield of α(1,2) galactose incorporated into polymannose.

### Example 8. Addition of beta (1,3) galactose to surface α(1,2) galactose residues

Next, β(1,3) galactose is added to the α(1,2) galactose previously incorporated in Example 7 (above). *Schizosaccharomyces pombe* possesses an enzyme well suited to this purpose, a β(1,3) galactosyltransferase that is the product of the PVG3 gene (Andreishcheva E.N. et al., 2004 J Biol Chem, 279:35644-35655). This gene is introduced into the engineered *K. lactis* strain from Example 7. Figure 15, lane 3, demonstrates expression of MYC-tagged *S.pombe* PVG3 in *K.lactis,* utilizing using an expression cassette, vector and strain as outlined in Example 4. Successful incorporation of galactose into *K. lactis* mannan is monitored using lectin-binding assays. *(e.g., Griffonia simplificolia* Lectin I-B₄ binding for α-linked galactose, Peanut agglutinin binding for beta-linked galactose).

### Example 9. Expression of human H epitopes on the K. lactis cell surface and optimization of H-epitope levels

H-antigen is expressed on *K. lactis* cell surface by the addition of α(1,2) fucosyl-groups to the β(1,3) galactose-modified mannan of the strain generated in Example 8. α(1,2) fucosylation is accomplished in humans by either one of two type II transmembrane Golgi α(1,2) fucosyltransferases that are the products of two distinct but related genes, Homo sapiens fucosyltransferase 1 (galactoside 2-alpha-L-fucosyltransferase; FUT1) and Homo sapiens fucosyltransferase 2 (FUT2) (Sarnesto A. et al., 1992 J Biol Chem, 267:2737-2744; Larsen R.D. et al., 1990 Proc Natl Acad Sci U S A, 87, 6674-6678; Kelly R.J. et al., 1995 J Biol Chem, 270:4640-4649). FUT1 (also known as the H-gene) is expressed in human mesenchymal tissues; FUT2 (also known as the secretor gene or Se) is expressed in human epithelial tissues. FUT2 is responsible for α(1,2) fucosylation of glycans in secretions (tears, saliva, milk etc) and on epithelial/mucosal surfaces, and is the enzyme responsible for the fucosylation that has been shown to protect against pathogen adherence in humans. FUT1 and FUT2 are only 68% identical at the amino acid level across their catalytic domains; however they have been directly demonstrated to be quite similar enzymatically in terms of substrate preference, despite earlier studies that had predicted that substantial differences between them might exist. Each enzyme is tested individually in *K. lactis,* with H-antigen expression being monitored by *Ulex europaeus* lectin binding or by ELISA. FUT1 and FUT2 possess minor difference substrate specificity. Each or both gene products may be expressed and the level or ratio of expression manipulated to maximize final yields of H-antigen. Figure 15, lane 4, demonstrates expression of MYC-tagged human FUT1 in *K.lactis,* utilizing using an expression cassette, vector and strain as outlined in Example 4.

Several additional approaches are optionally utilized to yield optimization.
- use of *mnn1* and *mnn2* mutants (see examples 6 and 7 above)
- use of VRG4 mutants (see example 4 above)
- use of phosphomannose isomerase (PMI), phosphomannose mutase (PMM) and GDP-mannose pyrophosphorylase (PSA1) over-expression (see example 4 above)
- tailoring of enzyme levels through control of expression parameters, e.g. the use of stringer or weaker promoters in the expression cassettes described in example 4 (above)
- Cell yields are optimized under controlled conditions in a bioreactor by manipulating variables such as base growth medium, carbon source, inoculum preparation procedure, and various physical growth conditions (i.e., aeration/agitation rate, culture temperature, culture pH). Statistical methods for rapid optimization of multiple variables in fermentation processes are well established and are employed here (Kalil S.J. et al., 2001 Appl Biochem Biotechnol, 94:257-264).

### Example 10. Biological Testing

The engineered strain of *K. lactis* is used as a probiotic product. The engineered strain carries on its cell surface polyvalent α(1,2) fucosylated glycan structures that bind efficiently to adherence determinants of a number of pathogenic organisms. The engineered strain when ingested acts as a decoy for gut pathogens. The ability of the surface α(1,2) fucosylated *K. lactis* strain generated in this invention prevents adherence and infection by pathogens. The *K. lactis* strain is tested for efficacy in suitable animal models of gut infection (Newell D.G., 2001 Symp Ser Soc Appl Microbiol, 57S-67S).

### Direct binding of Campylobacter to engineered K. lactis cells

The ability of *Campylobacter* to adhere to engineered *K. lactis* is assessed microscopically. Wild type and engineered *K lactis* are incubated with bacteria, washed, and bound bacteria visualized and quantified under a phase contrast microscope.

### Solid-phase assays

The ability of *Campylobacter* to adhere to histo-blood group antigens, and the inhibition of this binding by various agents, is assessed in bacterial-binding Western blot assays using DIG-labeled bacteria. Mucin is run on lanes of SDS-PAGE gels and then electro-blotted onto nitrocellulose membranes. Membranes are then washed, immersed in a DIG-labeled bacterial suspension, and bound bacteria visualized with alkaline phosphatase-conjugated anti-DIG antibody stained with X-Phosphate and nitroblue tetrazolium. In a solid-phase Norwalk Virus binding inhibition assay, saliva obtained from secretors is purified and placed at the bottom of a 96-well plate. The binding of Norwalk Virus capsid is measured using known methods. The effectiveness of test agents in inhibiting adherence in both assays is assessed readily. In the case of the engineered *K. lactis* strain generated here, the effectiveness of a pre-incubation step with pathogen to reduce the observed signal in each assay is assessed.

### Mammalian cell-binding assay

α(1,2) fucosylated glycans generated as described herein are tested for their ability to inhibit binding of *Campylobacter* to genetically modified CHO cells. Bacterial binding, in the presence or absence of a pre-incubation step with engineered *K. lactis,* to CHO cells transfected with human α(1,2) fucosyltransferase (*FUT1*) and expressing cell-surface α(1,2) fucosylated proteins, is compared to vector only-transfected and parental CHO cells. Data are interpreted as percent inhibition of bacteria association to cells relative to positive controls.

### In vivo testing

*Campylobacter* colonization *in vivo* is achieved in the inbred mouse strain, BALB/c (Blaser M.J. et al., 1983 Infect Immun, 39:908-916). Inhibition or reduction of infection by the surface α(1,2)-fucosylated *K. lactis* strain generated as described herein is assessed in two modes, *i.e.,* by prophylaxis and by post-infection treatments. Engineered yeast, wild type yeast or vehicle are administered to mice p.o., either 2d prior or 7d subsequent to inoculation with 10⁸ CFU of the *Campylobacter jejuni* invasive strain 287ip. Levels of colonization are monitored by measuring *Campylobacter* CFU in stool samples.

## Claims

1. A composition comprising a yeast cell, said yeast cell comprising an exogenous nucleic acid molecule encoding GDP-mannose-4,6-dehydratase (GMD) and an exogenous nucleic acid molecule encoding GDP-L-fucose synthetase (GFS), wherein said yeast cell further comprises a nucleic acid molecule encoding GDP-mannose pyrophosphorylase, phosphomannose isomerase, or phosphomannose mutase.

2. The composition of claim 1, wherein said GMD is selected from the group consisting of *H. pylori* GMD, *E. coli* GMD, and *Homo sapiens* GMD.

3. The composition of claim 1, wherein said yeast cell further harbors a nucleic acid molecule encoding a nucleotide sugar transporter (NST), preferably wherein said NST is selected from the group consisting of human FUCT1 , human UGT1, and *S.cerevisiae* HUT1.

4. The composition of claim 1, wherein said yeast cell over-expresses an endogenous NST.

5. The composition of claim 3, wherein said yeast cell lacks terminal *N-*acetylglucosaminyltransferase activity or UDP-*N*-acetylglucosamine transporter activity.

6. The composition of claim 1, wherein said yeast cell is a *Kluyveromyces lactis* yeast cell, preferably wherein said yeast cell is a *Kluyveromyces lactis* yeast cell carrying a *mnn2-1* or *mnn2-2* mutation.

7. The composition of claim 5, wherein said yeast cell further harbors a nucleic acid molecule encoding an α(1,2) galactosyltransferase, preferably wherein said nucleic acid molecule encoding an α(1,2) galactosyltransferase is *Schizosaccharomyces pombe gma12.*

8. The composition of claim 7, wherein said yeast cell further harbors a nucleic acid molecule encoding a β(1,3) galactosyltransferase, preferably wherein said nucleic acid molecule encoding a β(1,3) galactosyltransferase is *Schizosaccharomyces pombe* PVG3.

9. The composition of claim 7, wherein said yeast cell further harbors a recombinant nucleic acid molecule encoding a fucosyltransferase, preferably wherein said fucosyltransferase is a human α(1,2) fucosyltransferase.

10. The composition of claim 1, wherein the genus of said yeast cell is *Kluyveromyces.*

11. A composition comprising a yeast cell that displays a non-native glycan on its cell surface, wherein said non-native glycan comprises an α fucosylated glycan or a sialyl glycan.

12. The composition of claim 11, wherein the genus of said yeast cell is *Kluyveromyces* or *Saccharomyces.*

13. The composition of claim 11, wherein said yeast cell is a *Kluyveromyces lactis* yeast cell.

14. A method for producing yeast containing GDP-fucose comprising culturing the yeast of claim 1 so that said encoded GDP-mannose-4,6-dehydratase and said encoded GDP-L-fucose synthetase are expressed.

15. A method for producing yeast containing GDP-fucose comprising culturing the yeast of claim 1 so that said encoded GDP-mannose-4,6-dehydratase and said encoded GDP-L-fucose synthetase are expressed simultaneously with at least one overexpressed enzyme selected from the group consisting ofphosphomannose isomerase, phosphomannose mutase, and GDP-mannose pyrophosphorylase.

16. A method for producing yeast that are competent for the Golgi import of both GDP-fucose and UDP-galactose comprising culturing the yeast of claim 3 so that said encoded nucleotide sugar transporter is expressed.

17. A method for producing yeast lacking terminal α(1,2) GlcNAc mannans comprising introducing the *Kluyveromyces lactis* yeast cell *mnn2-1* or *mnn2-2* mutation into the yeast cell of claim 3.

18. A method of producing yeast with α(1,2) galactose residues on surface polymannose, comprising culturing the yeast of claim 7 so that the encoded α(1,2) glycosyltransferase is expressed.

19. A method of producing yeast with β(1,3) galactose linked to surface α(1,2) galactose residues comprising culturing the yeast of claim 8 so that the β(1,3) glycosyltransferase is expressed.

20. A method of producing yeast with α(1,2) fucosylated glycans on its cell surface comprising culturing the yeast of claim 9 so that the fucosyltransferase is expressed.

21. The composition of claim 11, wherein said glycan binds to a pathogen for use in treating, preventing, or reducing the risk of infection in a subject, wherein said subject is infected with or at risk of infection with said pathogen, wherein the composition is for administration to said subject.

22. The composition for use according to claim 21, wherein said infection is caused by a Norwalk-like virus or *Campylobacter jejuni.*

23. The composition for use according to claim 21, wherein said subject is a mammal, preferably a human, pig, horse, chicken, cow, sheep, goat, dog, or cat.

24. The composition of claim 21, wherein said composition inhibits or reduces binding of said pathogen to a cell of said subject.

## Patentansprüche

1. Zusammensetzung, die eine Hefezelle umfasst, wobei die Hefezelle ein exogenes Nukleinsäuremolekül, das GDP-Mannose-4,6-dehydratase (GMD) kodiert, und ein exogenes Nukleinsäuremolekül, das GDP-L-Fucose-Synthetase (GFS) kodiert, umfasst, wobei die Hefezelle ferner ein Nukleinsäuremolekül umfasst, das GDP-Mannosepyrophosphorylase, Phosphomannoseisomerase oder Phosphomannosemutase kodiert.

2. Zusammensetzung nach Anspruch 1, wobei die GMD aus der Gruppe, bestehend aus *H. pylori* GMD, *E coli* GMD und *Homo sapiens* GMD, ausgewählt ist.

3. Zusammensetzung nach Anspruch 1, wobei die Hefezelle ferner ein Nukleinsäuremolekül enthält, das einen Nukleotidzuckertransporter (NST) kodiert, wobei der NST vorzugsweise aus der Gruppe, bestehend aus menschlichem FUCT1, menschlichem UGT1 und *S. cerevisiae* HUT1, ausgewählt ist.

4. Zusammensetzung nach Anspruch 1, wobei die Hefezelle einen endogenen NST überexprimiert.

5. Zusammensetzung nach Anspruch 3, wobei der Hefezelle eine entständige N-Acetylglucosaminyltransferaseaktivität oder UDP-*N*-acetylglukosamintransporteraktivität fehlt.

6. Zusammensetzung nach Anspruch 1, wobei die Hefezelle eine *Kluyveromyces lactis-*Hefezelle ist, wobei die Hefezelle vorzugsweise eine *Kluyveromyces lactis*-Hefezelle ist, die eine *mnn2-1-* oder *mnn2-2*-Mutation aufweist.

7. Zusammensetzung nach Anspruch 5, wobei die Hefezelle ferner ein Nukleinsäuremolekül enthält, das eine α(1,2)-Galactosyltransferase kodiert, wobei das Nukleinsäuremolekül, das eine α(1,2)-Galactosyltransferase kodiert, vorzugsweise *Schizosaccharomyces pombe gma 12* ist.

8. Zusammensetzung nach Anspruch 7, wobei die Hefezelle ferner ein Nukleinsäuremolekül enthält, das eine β(1,3)-Galactosyltransferase kodiert, wobei das Nukleinsäuremolekül, das eine β(1,3)-Galactosyltransferase kodiert, vorzugsweise *Schizosaccharomyces pombe PVG3* ist.

9. Zusammensetzung nach Anspruch 7, wobei die Hefezelle ferner ein rekombinantes Nukleinsäuremolekül enthält, das eine Fucosyltransferase kodiert, wobei die Fucosyltransferase vorzugsweise eine menschliche α(1,2)-Fucosyltransferase ist.

10. Zusammensetzung nach Anspruch 1, wobei die Gattung der Hefezelle *Kluyveromyces* ist.

11. Zusammensetzung, die eine Hefezelle umfasst, die ein nicht-natives Glycan auf ihrer Zelloberfläche präsentiert, wobei das nicht-native Glycan ein α-fucosyliertes Glycan oder ein Sialylglycan umfasst.

12. Zusammensetzung nach Anspruch 11, wobei die Gattung der Hefezelle *Kluyveromyces* oder *Saccharomyces* ist.

13. Zusammensetzung nach Anspruch 11, wobei die Hefezelle eine *Kluyveromyces lactis*-Hefezelle ist.

14. Verfahren zur Herstellung einer Hefe, die GDP-Fucose enthält, wobei das Verfahren das Kultivieren der Hefe nach Anspruch 1 umfasst, so dass die kodierte GDP-Mannose-4,6-dehydratase und die kodierte GDP-L-Fucose-Synthetase exprimiert werden.

15. Verfahren zur Herstellung einer Hefe, die GDP-Fucose enthält, wobei das Verfahren das Kultivieren der Hefe nach Anspruch 1 umfasst, so dass die kodierte GDP-Mannose-4,6-dehydratase und die kodierte GDP-L-Fucose-Synthetase gleichzeitig mit mindestens einem überexprimierten Enzym exprimiert werden, das aus der Gruppe, bestehend aus Phosphomannoseisomerase, Phosphomannosemutase und GDP-Mannosepyrophosphorylase, ausgewählt ist.

16. Verfahren zur Herstellung einer Hefe, die für die Golgi-Einführung sowohl von GDP-Fucose als auch von UDP-Galactose kompetent ist, umfassend das Kultivieren der Hefe nach Anspruch 3, so dass der kodierte Nukleotidzuckertransporter exprimiert wird.

17. Verfahren zur Herstellung einer Hefe, der entständige α(1,2)-GlcNAc-Mannane fehlen, umfassend das Einbringen der *Kluyveromyces lactis*-Hefezelle-*mnn2-1*- oder *mnn2-2*-Mutation in die Hefezelle nach Anspruch 3.

18. Verfahren zur Herstellung einer Hefe mit α(1,2)-Galactoseresten auf Oberflächenpolymannose, umfassend das Kultivieren der Hefe nach Anspruch 7, so dass die kodierte α(1,2)-Glycosyltransferase exprimiert wird.

19. Verfahren zur Herstellung einer Hefe mit β(1,3)-Galactose, die mit Oberflächen-α(1,2)-Galactoseresten verknüpft ist, umfassend das Kultivieren der Hefe nach Anspruch 8, so dass die β(1,3)-Glycosyltransferase exprimiert wird.

20. Verfahren zur Herstellung einer Hefe mit α(1,2)-fucosylierten Glycanen auf deren Zelloberfläche, umfassend das Kultivieren der Hefe nach Anspruch 9, so dass die Fucosyltransferase exprimiert wird.

21. Zusammensetzung nach Anspruch 11, wobei das Glycan an ein Pathogen bindet, zur Verwendung bei der Behandlung, Prävention oder Verminderung des Risikos einer Infektion in einem Lebewesen, wobei das Lebewesen mit dem Pathogen infiziert ist oder bei dem Lebewesen das Risiko einer Infektion mit dem Pathogen besteht, wobei die Zusammensetzung zur Verabreichung an das Lebewesen dient.

22. Zusammensetzung zur Verwendung nach Anspruch 21, wobei die Infektion durch ein Norwalk-artiges Virus oder *Campylobacter jejuni* verursacht wird.

23. Zusammensetzung zur Verwendung nach Anspruch 21, wobei das Lebewesen ein Säuger, vorzugsweise ein Mensch, ein Schwein, ein Pferd, ein Huhn, eine Kuh, ein Schaf, eine Ziege, ein Hund oder eine Katze ist.

24. Zusammensetzung nach Anspruch 21, wobei die Zusammensetzung das Binden des Pathogens an eine Zelle des Lebewesens hemmt oder vermindert.

## Revendications

1. Composition comprenant une cellule de levure, ladite cellule de levure comprenant une molécule d'acide nucléique exogène codant la GDP-mannose-4,6-déshydratase (GMD) et une molécule d'acide nucléique exogène codant la GDP-L-fucose synthétase (GFS), dans laquelle ladite cellule de levure comprend en outre une molécule d'acide nucléique codant la GDP-mannose pyrophosphorylase, la phosphomannose isomérase, ou la phosphomannose mutase.

2. Composition selon la revendication 1, dans laquelle ladite GMD est choisie dans l'ensemble constitué par la GMD de *H. pylori,* la GMD d'*E*. *coli,* et la GMD de *Homo sapiens.*

3. Composition selon la revendication 1, dans laquelle ladite cellule de levure héberge en outre une molécule d'acide nucléique codant un transporteur de sucre nucléotidique (NST), de préférence dans laquelle ledit NST est choisi dans l'ensemble constitué par le FUCT1 humain, l'UGT1 humain, et le HUT1 de *S*. *cerevisiae.*

4. Composition selon la revendication 1, dans laquelle ladite cellule de levure surexprime un NST endogène.

5. Composition selon la revendication 3, dans laquelle ladite cellule de levure est dépourvue d'activité de N-acétylglucosaminyltransférase terminale ou d'activité de transporteur d'UDP-N-acétylglucosamine.

6. Composition selon la revendication 1, dans laquelle ladite cellule de levure est une cellule de levure de *Kluyveromyces lactis,* de préférence dans laquelle ladite cellule de levure est une cellule de levure de *Kluyveromyces lactis* portant une mutation mnn2-1 *ou mnn2-2.*

7. Composition selon la revendication 5, dans laquelle ladite cellule de levure héberge en outre une molécule d'acide nucléique codant une α(1,2) galactosyltransférase, de préférence dans laquelle ladite molécule d'acide nucléique codant une α(1,2) galactosyltransférase est *Schizosaccharomyces pombe gma12.*

8. Composition selon la revendication 7, dans laquelle ladite cellule de levure héberge en outre une molécule d'acide nucléique codant une β(1,3) galactosyltransférase, de préférence dans laquelle ladite molécule d'acide nucléique codant une β(1,3) galactosyltransférase est *Schizosaccharomyces pombe* PVG3.

9. Composition selon la revendication 7, dans laquelle ladite cellule de levure héberge en outre une molécule d'acide nucléique recombiné codant une fucosyltransférase, de préférence dans laquelle ladite fucosyltransférase est une α(1,2) fucosyltransférase humaine.

10. Composition selon la revendication 1, dans lequel le genre de ladite cellule de levure est *Kluyveromyces.*

11. Composition comprenant une cellule de levure qui présente un glycane non natif sur sa surface cellulaire, dans laquelle ledit glycane non natif comprend un glycane α-fucosylé ou un sialyl-glycane.

12. Composition selon la revendication 11, dans laquelle le genre de ladite cellule de levure est *Kluyveromyces* ou *Saccharomyces.*

13. Composition selon la revendication 11, dans laquelle ladite cellule de levure est une cellule de levure de *Kluyveromyces lactis.*

14. Procédé pour produire une levure contenant du GDP-fucose, comprenant la culture de la levure selon la revendication 1 de sorte que ladite GDP-mannose-4,6-déshydratase codée et ladite GDP-L-fucose synthétase codée soient exprimées.

15. Procédé pour produire une levure contenant du GDP-fucose, comprenant la culture de la levure selon la revendication 1 de sorte que ladite GDP-mannose-4,6-déshydratase codée et ladite GDP-L-fucose synthétase codée soient exprimées simultanément avec au moins une enzyme surexprimée choisie dans l'ensemble constitué par la phosphomannose isomérase, la phosphomannose mutase, et la GDP-mannose pyrophosphorylase.

16. Procédé pour produire une levure compétente pour l'importation dans le complexe de Golgi à la fois de GDP-fucose et d'UDP-galactose, comprenant la culture de la levure selon la revendication 3 de sorte que ledit transporteur de sucre nucléotidique codé soit exprimé.

17. Procédé pour produire une levure dépourvue d'α(1,2) GlcNAc mannanes terminaux, comprenant l'introduction de la mutation *mmn2-1* ou mnn2-2 de cellule de levure de *Kluyveromyces lactis* dans la cellule de levure selon la revendication 3.

18. Procédé pour produire une levure avec des résidus α(1,2) galactose sur du polymannose de surface, comprenant la culture de la levure selon la revendication 7 de sorte que l'α(1,2) glycosyltransférase codée soit exprimée.

19. Procédé pour produire une levure avec du β(1,3) galactose lié à des résidus α(1,2) galactose de surface, comprenant la culture de la levure selon la revendication 8 de sorte que la β(1,3) glycosyltransférase soit exprimée.

20. Procédé pour produire une levure avec des glycanes α(1,2) fucosylés sur sa surface cellulaire, comprenant la culture de la levure selon la revendication 9 de sorte que la fucosyltransférase soit exprimée.

21. Composition selon la revendication 11, dans laquelle ledit glycane se lie à un pathogène, pour utilisation dans le traitement, la prévention, ou la réduction du risque d'une infection chez un sujet, dans laquelle ledit sujet est infecté ou risque d'être infecté par ledit pathogène, laquelle composition est destinée à être administrée audit sujet.

22. Composition pour utilisation selon la revendication 21, dans laquelle ladite infection est due à un virus de type Norwalk ou à *Campylobacter jejuni.*

23. Composition pour utilisation selon la revendication 21, dans laquelle ledit sujet est un mammifère, de préférence un humain, un cochon, un cheval, un poulet, une vache, un mouton, une chèvre, un chien, ou un chat.

24. Composition pour utilisation selon la revendication 21, laquelle composition inhibe ou réduit la liaison dudit pathogène à une cellule dudit sujet.
